(19) Europäisches Patentamt
European Patent Office
Office européen des brevets

(11) **EP 4 717 710 A1**

(12) **EUROPEAN PATENT APPLICATION**
published in accordance with Art. 153(4) EPC

(43) Date of publication:
01.04.2026 Bulletin 2026/14

(21) Application number: 24811394.6

(22) Date of filing: 22.05.2024

(51) International Patent Classification (IPC):
C07K 16/28 (2006.01)    A61P 29/00 (2006.01)
A61P 37/00 (2006.01)    A61P 11/02 (2006.01)
A61K 39/00 (2006.01)

(52) Cooperative Patent Classification (CPC):
A61K 39/00; A61P 11/02; A61P 29/00; A61P 37/00;
C07K 16/28

(86) International application number:
PCT/KR2024/006903

(87) International publication number:
WO 2024/242455 (28.11.2024 Gazette 2024/48)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 23.05.2023 KR 20230066415

(71) Applicants:
• UIF (University Industry Foundation), Yonsei
University
Seoul 03722 (KR)
• Hongik University Industry-Academia
Cooperation Foundation
Mapo-gu, Seoul 04066 (KR)

(72) Inventors:
• KIM, Joo Young
Goyang-si Gyeonggi-do 10394 (KR)
• CHO, Hyungju
Seoul 06518 (KR)
• AHN, Taeyoung
Seoul 03759 (KR)
• PARK, Joon-Sang
Seoul 04132 (KR)

(74) Representative: Mewburn Ellis LLP
Aurora Building
Counterslip
Bristol BS1 6BX (GB)

Remarks:
The complete document including Reference
Table(s) and the Sequence Listing(s) can be
downloaded from the EPO website

(54) **NOVEL HUMAN INTERLEUKIN-4 RECEPTOR-BINDING NANOBODY AND NASAL SPRAY FORMULATION COMPRISING SAME**

(57) The present invention relates to antigen-binding fragments, more specifically nanobodies, that exhibit remarkable binding affinity for the IL-4 receptor, thereby effectively blocking IL-4-mediated signal transduction, and that are capable of directly penetrating epithelial cells due to their low molecular weight. The present inventors have further identified amino acid substitution variants and bivalent forms thereof that maximize the IL-4 receptor-binding affinity of the nanobodies and confirmed that these variants exhibit excellent nasal epithelial cell permeability and significant therapeutic effects on inflammatory responses. Accordingly, the present invention may be usefully applied as a composition for the treatment of chronic inflammatory diseases, particularly as a nasal spray formulation that avoids the various adverse effects associated with systemic administration.

EP 4 717 710 A1

**FIG. 8**

## Description

### Technical Field

The present invention relates to a nanobody that specifically binds to the IL-4 receptor, a nasal spray comprising the nanobody as an active ingredient and a method of preventing or treating various IL-4-mediated inflammatory diseases using the nanobody as a pharmaceutical ingredient.

### Background Art

The hollow spaces within the facial bones around the nose are called paranasal sinuses, and these spaces are connected to the inside of the nose through tiny openings called natural ostia, which allow for the ventilation of air and the excretion of secretions. sinuses, is a condition in which the natural ostia become obstructed, resulting in inadequate ventilation and drainage of the paranasal sinuses and thereby causing secondary inflammation of the sinuses, with the inflammation progressively worsening as purulent secretions accumulate within the sinuses. This sinusitis is defined as chronic rhinosinusitis (CRS) when it persists for more than three months.

Among cases of chronic rhinosinusitis, those accompanied by nasal polyps account for up to 33% of all sinusitis cases, are associated with various respiratory diseases, and are known to have a poor prognosis even after endoscopic sinus surgery.

Dupilumab, a monoclonal antibody against interleukin-4 receptor alpha (IL-4R$\alpha$), has been shown to be effective in the treatment of chronic rhinosinusitis (CRS). However, due to the molecular size of the antibody, direct administration to the affected sinus tissue is not possible, and it is currently available only as an injectable formulation, which is associated with various adverse effects including injection site reactions and serum sickness like reactions. "Nanobodies" refer to the antigen-recognizing variable region of heavy chain-only antibodies found in camelids. Due to their small molecular weight and high production yield, nanobodies can address various problems associated with conventional antibody therapeutics including limitation in routes of administration.

On the other hand, AI-based in silico affinity maturation technology predicts the structure of an antigen-binding fragment such as a nanobody and identifies its binding structure to a receptor, and then discovers mutations that can increase binding ability to the receptor through computer program-based affinity enhancement techniques, which is very useful for maximizing the ability of the nanobody to bind to the receptor.

Accordingly, the present inventors sought to discover a novel IL-4R inhibitory antibody that exhibits a markedly higher IL-4 receptor inhibitory effect and high stability compared to dupilumab. In addition, due to its small molecular weight, the antibody can be administered in the form of a spray directly to nasal epithelial cells to pass through the epithelial layer and reduce inflammation, and the inventors further sought to develop a mutant antibody with maximized binding affinity through a computer program-based affinity maturation technique.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to address the problems of conventional anti-IL-4R antibodies, which exhibit excellent therapeutic effects on various inflammatory diseases including sinusitis but cannot pass through epithelial cells due to their large molecular weight and therefore must be administered only by injection, leading to various adverse effects. As a result, the present inventors have successfully identified antigen-binding fragments comprising the amino acid sequences of SEQ ID NOs: 1 and 5; and nanobodies comprising amino acid substitution mutations that enhance receptor-binding affinity. The present inventors further confirmed that, when formulated as a nasal spray and locally administered to inflamed paranasal sinus tissue, the nanobodies can prevent or treat inflammation without adverse effects, thereby completing the present invention.

Accordingly, it is an object of the present invention to provide antibodies to the IL-4 receptor (IL-4R) or antigen-binding fragments thereof and nucleic acid molecules encoding the same.

It is another object of the present invention to provide pharmaceutical compositions for preventing or treating inflammatory diseases or autoimmune diseases that are also applicable as nasal spray formulations.

Other objects and advantages of the present invention will become more apparent from the following detailed description, the appended claims, and the accompanying drawings.

**Technical Solution**

In one aspect of this invention, there is provided an anti-IL-4R antibody or antigen-binding fragment thereof comprising the amino acid sequence of SEQ ID NO: 1, wherein the anti-IL-4R antibody or antigen-binding fragment thereof comprises substitutions of one or more amino acid residues selected from the group consisting of the 5th residue and the 11th residue in the amino acid sequence of SEQ ID NO: 1.

The present inventors have made intensive studies to develop address the problems of conventional anti-IL-4R antibodies, which have excellent therapeutic effects on various inflammatory diseases including sinusitis as IL-4 receptor inhibitors, but cannot pass through epithelial cells due to their large molecular weight and therefore must be administered only by injection, resulting in various adverse effects. As a result, the present inventors have successfully identified antigen-binding fragments comprising the amino acid sequences of SEQ ID NOs: 1 and 5 and nanobodies comprising amino acid substitution mutations that further enhance receptor-binding affinity. The present inventors further discovered that when formulated as a nasal spray and directly administered to inflamed sinus tissue, the nanobodies can efficiently prevent or treat inflammation without adverse effects.

As used herein, the term "antibody" refers to a peptide molecule that that specifically recognizes and binds to a specific epitope of IL-4, and includes antigen-binding fragments of the antibody (immunologically active fragments) as well as the whole antibody molecule.

A complete antibody has a structure with two full-length light chains and two full-length heavy chains, which are linked to each other by a disulfide bond. The heavy chain constant region has the gamma ($\gamma$), mu ($\mu$), alpha ($\alpha$), delta ($\delta$), and epsilon ($\epsilon$) types and is subclassified into gammal ($\gamma$1), gamma2 ($\gamma$2), gamma3 ($\gamma$3), gamma4 ($\gamma$4), alpha1 ($\alpha$1) and alpha2 ($\alpha$2). The light chain constant region has kappa ($\kappa$) and lambda ($\lambda$) types.

As used herein, the term "antigen-binding fragment of the antibody" refers to a portion of a polypeptide that has significant antigen-antibody binding functionality within the overall structure of an immunoglobulin. Examples of the antigen-binding fragment include Fab, F(ab'), F(ab')2, Fv and nanobody (sybody).

Among antibody fragments, Fab is a structure with one antigen-binding site containing variable regions of the light and heavy chains, a constant region of the light chain and the first constant region of the heavy chain (CH1). Fab' differs from Fab in that it has a hinge domain containing one or more cysteine residues at the C-terminus of the heavy chain CH1 domain. F(ab')2 antibodies are generated by disulfide bond formed by cysteine residues in the hinge of Fab'. Recombinant techniques for generating Fv fragments with minimal antibody fragments that have only a heavy chain variable region and a light chain variable region are disclosed in WO88/10649, WO88/106630, WO88/07085, WO88/07086, and WO88/09344. Two-chain Fv has a heavy chain variable region and a light chain linked by non-covalent bonds, while single-chain Fv has a heavy chain variable region and a light chain variable region linked by covalent bonds, usually via a peptide linker, or directly at the C-terminus, which can form a dimer-like structure. Such antibody fragments could be obtained by proteolytic digestion of whole antibodies (for example, Fab fragments can be produced by papain digestion and F(ab')2 fragments can be produced by pepsin digestion), and could also be produced by recombinant genetic engineering techniques.

As used herein, the term "heavy chain" refers to both full-length heavy chain and fragments thereof comprising a variable region VH and three constant regions CH1, CH2 and CH3 with the amino acid sequence having sufficient variable region sequence to confer specificity to an antigen.

As used herein, the term "light chain" refers to both full-length light chain and fragments thereof comprising a variable region VL and a constant region CL with the amino acid sequence having sufficient variable region sequence to confer specificity to an antigen.

As used herein, the term "complementarity determining region (CDR)" refers to the amino acid sequence of the hypervariable region of the heavy and light chains of an immunoglobulin. The heavy chain (HCDR1, HCDR2, and HCDR3) and light chain (LCDR1, LCDR2, and LCDR3) each contain three CDRs, which provide the primary contact residues for an antibody to bind to an antigen or epitope. The scope of the antibody or antigen-binding fragment of the present invention includes variants having conservative amino acid substitutions in the CDR region. Furthermore, the antibody or antigen-binding fragment of the present invention includes variants of the amino acid in the attached sequence listings, to the extent that they are capable of specifically recognizing NKp46. For example, additional changes can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

In addition, amino acid substitutions in proteins that do not alter the activity of the molecule as a whole are known in the art (H. Neurath, R.L. Hill, The Proteins, Academic Press, New York, 1979). The most common exchanges are between the amino acid residues Ala/Ser, Val/Ile, Asp/Glu, Thr/Ser, Ala/Gly, Ala/Thr, Ser/Asn, Ala/Val, Ser/Gly, Thr/Phe, Ala/Pro, Lys/Arg, Asp/Asn, Leu/Ile, Leu/Val, Ala/Glu, and Asp/Gly.

Considering biologically equivalent variations, the amino acid sequence constituting the antibody of the present invention may encompass sequences having substantial identity to them. Sequences having the substantial identity show at least 61%, specifically at least 70%, more specifically at least 80%, even more specifically at least 90% similarity to the amino acid of this invention, as measured using one of the sequence comparison algorithms. Methods of alignment of sequences for comparison are well-known in the art. Various methods and algorithms for alignment are disclosed in Huang et al., Comp. Appl. BioSci. 8:155-65(1992) and Pearson et al., Meth. Mol. Biol. 24:307-31(1994).

As used herein, the term "IL-4" refers to a cytokine that induces the differentiation of unactivated helper T cells (naive T cells, Th0 cells) into Th2 cells. IL-4 has various biological functions, including stimulating activated B cells and T cells and inducing the differentiation of B cells into plasma cells, and serves as an important regulator of humoral and acquired immunity.

As used herein, the term "IL-4R" refers to the receptor for IL-4, which induces signaling associated with IgE antibody production upon binding to its ligand, IL-4. The receptor for IL-4 is generally known as IL-4R$\alpha$ and exists in the body in two complex forms. Type 1 receptors generally exist as a complex of the $\gamma$ chain ($\gamma$c) and IL-4R$\alpha$, and are specific to IL-4. Type 2 receptors generally exist as a complex of IL-4R$\alpha$ and IL-13R$\alpha$1, and are specific to both IL-4 and IL-13.

As used herein, the term "dupilumab" refers to a monoclonal antibody against IL-4R$\alpha$, which is an antibody therapeutic commercially available under the product name DUPIXENT®. Dupilumab's mechanism of action is known to involve inhibition of signal transduction at the Type 1 IL-4 receptor by blocking the binding of IL-4 to IL-4R$\alpha$ or by inhibiting the dimerization of IL-4R$\alpha$ and the $\gamma$ chain ($\gamma$c), and inhibition of signal transduction at the Type 2 IL-4 receptor by inhibiting the dimerization of IL-4R$\alpha$ and IL-13R$\alpha$1.

The antibodies of the present invention include, but are not limited to, monoclonal antibodies, human antibodies, humanized antibodies, chimeric antibodies, single-chain variable fragments (scFvs), single-chain antibodies, Fab fragments, F(ab') fragments, disulfide-bonded Fvs (sdFvs), anti-idiotype (anti-Id) antibodies, and epitope-binding fragments and nanobody (sybody) derived from the antibodies described herein

As used herein, the term "monoclonal antibody" refers to an antibody molecule of a single molecular composition obtained from a substantially homogeneous antibody population, wherein the monoclonal antibody exhibits a single binding specificity and affinity for a specific epitope.

As used herein, the term "nanobody" refers to a single-domain antibody analog comprising only one heavy chain variable region, and the is used interchangeably with the terms "single domain antibody (VHH antibody)" or "synthetic nanoantibody (sybody)". A nanobody is the smallest fully functional antigen-binding fragment, usually obtained by first isolating an antibody that naturally lacks the light chain and the first constant region (CH1) of the heavy chain, and then cloning the variable region of the heavy chain to construct a single-domain antibody (Variable Domain of Heavy-chain Antibodies, VHH) comprising only one heavy chain variable region. Nanobodies can be manufactured by artificially processing antibody fragments extracted from camels, llamas, alpacas and other mammalian animals, and have the advantages of being one-tenth the size of general antibodies, being resistant to external environments such as temperature changes, easy administration, and high production yield.

As used herein, the term "E5R" refers to a substitution mutation at position 5th of SEQ ID NO: 1 in which glutamic acid (E) is replaced with arginine (R).

As used herein, the term "A11Y" refers to a substitution mutation at position 11th of SEQ ID NO: 1 in which alanine (A) is replaced with tyrosine (Y).

According to a specific embodiment, the substitution of the 5th residue is a substitution to arginine (R), and the substitution of the 11th residue is a substitution to tyrosine (Y).

According to a specific embodiment, the anti-IL-4R antibody or antigen-binding fragment thereof comprises one or more amino acid sequences selected from the group consisting of SEQ ID NO: 2, SEQ ID NO: 3, and SEQ ID NO: 4.

In another aspect of this invention, there is provided an anti-IL-4R antibody or antigen-binding fragment thereof comprising the amino acid sequence of SEQ ID NO: 5, wherein the anti-IL-4R antibody or antigen-binding fragment thereof comprises substitutions of one or more amino acid residues selected from the group consisting of the 104th residue and the 110th residue in the amino acid sequence of SEQ ID NO: 5.

According to a specific embodiment, SEQ ID NO: 5 is an amino acid sequence of a full-length nanobody comprising a CDR sequence represented by SEQ ID NO: 1.

As used herein, the term "E104R" refers to a substitution mutation at position 104th of SEQ ID NO: 5 in which glutamic acid (E) is replaced with arginine (R).

As used herein, the term "A110Y" refers to a substitution mutation at position 110th of SEQ ID NO: 5 in which alanine (A) is replaced with tyrosine (Y).

According to a specific embodiment, the substitution of the 104th residue is a substitution to Arg, and the substitution of the 110th residue is a substitution to Tyr.

According to a specific embodiment, the anti-IL-4R antibody or antigen-binding fragment thereof comprises SEQ ID NO: 5 or SEQ ID NO: 6.

According to a specific embodiment, the anti-IL-4R antibody or antigen-binding fragment thereof comprises one or more amino acid sequences selected from the group consisting of SEQ ID NO: 6, SEQ ID NO: 7 and SEQ ID NO: 8.

According to a specific embodiment, the antigen-binding fragment is selected from the group consisting of F(ab')2, Fab', Fab, Fv, scFv and a Nanobody. More specifically, the antigen binding fragment is a Nanobody.

According to a specific embodiment, the nanobody comprises a convex scaffold structure.

As used herein, the term "convex scaffold" refers to the convex structural conformation of a nanobody. The CDR-H3 of a nanobody typically consists of approximately 19 amino acids, which is longer than the human CDR-H3 that typically consists of approximately 12 amino acids. This extended CDR-H3 forms a convex loop that enables effective binding to concave active sites of enzymes or proteases. For example, scFv, one of the antigen-binding fragments, binds well to flat linear epitopes, but nanobodies can also bind to concave active sites. However, nanobodies can also bind to flat active sites with high specificity, and this high affinity is attributed to the flexible scaffold of the nanobody. Furthermore, nanobodies have the advantage of exhibiting lower levels of nonspecific background binding compared to scFv.

In still another aspect of this invention, there is provided a dimeric antibody or antigen-binding fragment thereof comprising: two antibodies or antigen-binding fragments thereof that are identical or different from each other, wherein each antibody or antigen-binding fragment thereof is selected from the group consisting of SEQ ID NOs: 1, 2, 3, 4, 5, 6, 7 and 8; and a linker.

As used herein, the term "antibody or antigen-binding fragment in dimer form" refers to a bivalent nanobody in which two identical or different antibodies or antigen-binding fragments are linked.

According to a specific embodiment, the dimeric antibody or the antigen-binding fragment thereof consists of two antibodies or antigen-binding fragments thereof having the amino acid sequence of SEQ ID NO: 5 and a linker connecting the two antibodies or antigen-binding fragments thereof.

As used herein, the term "linker" refers to a means for connecting two different fusion partners (e.g., biological polymers) via covalent bonds. The linker may be a peptide linker or a non-peptide linker. When the linker is a peptide linker, it may comprise one or more amino acids.

As used herein, the term "peptide linker" refers to a linker composed of amino acids. The peptide linker may comprise from 1 to 60 amino acids, and more specifically, from 3 to 50 amino acids. However, the length of the linker is not particularly limited, and linkers of any length may be used, as long as they do not interfere with the receptor-binding ability of the bivalent nanobody. Any amino acid may be used to constitute the peptide linker, and the constituent peptides may be identical or different from each other. In a specific embodiment, the peptide linker may be an AAA linker.

According to a specific embodiment, the dimeric antibody or the antigen-binding fragment thereof consists of two antibodies or antigen-binding fragments thereof having the amino acid sequence of SEQ ID NO: 6 and a linker connecting the two antibodies or antigen-binding fragments thereof.

According to a specific embodiment, the dimeric antibody or the antigen-binding fragment thereof consists of two antibodies or antigen-binding fragments thereof having the amino acid sequence of SEQ ID NO: 7 and a linker connecting the two antibodies or antigen-binding fragments thereof.

According to a specific embodiment, the dimeric antibody or the antigen-binding fragment thereof consists of two antibodies or antigen-binding fragments thereof having the amino acid sequence of SEQ ID NO: 8 and a linker connecting the two antibodies or antigen-binding fragments thereof.

According to a specific embodiment, the linker consists of three amino acids. More specifically, the linker is a peptide having sequence of SEQ ID NO: 9.

In still another aspect of this invention, there is provided a nucleic acid molecule encoding the antibody or antigen-binding fragment thereof of the present invention.

As used herein, the term "nucleic acid molecule" is meant to encompass DNA (gDNA and cDNA) and RNA molecules. Nucleotides, which are the basic structural units in nucleic acid molecules, include not only natural nucleotides, but also analogues having modified sugar or base moieties (Uhlman and Peyman, Chemical Reviews, 90:543-584 (1990)). It will be clear to those skilled in the art that the nucleotide sequence encoding the amino acid sequence of a full-length antibody or a heavy chain, light chain variable region thereof, may involve modifications including additions, deletions, or non-conservative or conservative substitutions of nucleotides.

The nucleic acid molecule of the present invention is interpreted to include a nucleotide sequence that is substantially identical to any of the above nucleotide sequences. Substantial identity means a sequence showing at least 80% homology, specifically at least 90% homology, more specifically at least 95% homology as determined by aligning the sequence of the present invention with any other sequence to correspond to each other as much as possible and analyzing the aligned sequence using an algorithm commonly used in the art.

According to the present invention, the antibody or the antigen-binding fragment thereof of the present invention may be obtained recombinantly by expressing a nucleic acid molecule encoding it in a host cell.

As used herein, the term "express" refers to being artificially replicated as an extrachromosomal factor or by chromosomal integration in a target cell via a gene delivery system to cause the target cell to express an exogenous gene or overexpress an endogenous gene. Accordingly, "express" may be used interchangeably with "transformation", "transfection", or "transduction". More specifically, "to express" in the present invention refers to cause a target cell to artificially express an exogenous gene.

The term "gene delivery system" or "gene delivery vehicle" as used herein refers to any means for delivering a gene into a cell, and the term "gene delivery" has the same meaning as intracellular transduction of the gene. At the cell or tissue level, gene delivery has the same meaning as spread of the gene. Thus, the gene delivery system of the present invention may be referred to as a gene transduction system or a gene spread system.

To prepare a gene delivery vector of the present invention, the nucleotide sequence of the present invention is present in an appropriate expression construct, and, within the expression construct, the nucleotide sequence of the present invention may be operatively linked to an expression regulatory sequence. As used herein, the term "operatively linked" refers to a functional linkage between a nucleic acid expression regulatory sequence (e.g., a promoter, a signal sequence, or an array of transcription regulation factor binding sites) and the target nucleic acid sequence. Through the linkage, the regulatory sequence regulates the transcription and/or translation of the target nucleic acid sequence.

The gene delivery vector of the present invention may be constructed in various forms, including (i) a naked recombinant DNA molecule, (ii) a plasmid, (iii) a viral vector, and (iv) a liposome or niosome encapsulating the naked recombinant DNA molecule or the plasmid.

The nucleic acid molecule of the present invention may be used in the form of a gene therapy agent to deliver the antibody, antigen-binding fragment, or nanobody of the present invention in a genetic form to a subject, or may be used to produce a protein therapeutic by recombinantly expressing a modified antibody, antigen-binding fragment or nanobody in a host cell.

When mRNA is used as the nucleic acid molecule of the invention, various modifications can be made to improve the efficiency of expression (translation) of the antibody or the antigen-binding fragment, including, for example, changes in the length of the poly(A) tail or substitution of some adenine bases; modification of the 5'cap; application of one or more modified nucleosides. The modified nucleosides include, for example, N1-methylpseudouridine, pseudouridine, 2-thiouridine, 5-methyluridine, or 5-methylcytidine, 5-methylcytidine and 5-methoxyuridine, but are not limited thereto. Any modified nucleoside known in the art to reduce the immunogenicity of an mRNA molecule may also be used. When the present invention is used as an mRNA therapeutic for producing the antibody, antigen-binding fragment, or nanobody of the present invention in a patient, various lipid-based gene delivery systems (e.g., PEG-lipids, cholesterol, or ganglioside GM3) may be used to efficiently deliver the mRNA molecule.

According to a specific embodiment, the mRNA molecules of the present invention have a 5'-UTR and a 3'-UTR bound at both ends, respectively, and more specifically, said 5'-UTR is bound with a 5'-cap. As used herein, the term "untranslated region (UTR)" refers to an untranslated region within an mRNA that is bound at both ends of a coding sequence encoding a target protein, with a 5'-UTR located upstream of the coding sequence and a 3'-UTR located downstream of the coding sequence. The term "5'-cap" refers to a component of the mRNA that is linked to the 5'-UTR and binds to eIF4E (eukaryotic translation initiation factor 4E), thereby recruiting the 40S ribosomal subunit to the mRNA to initiate protein synthesis at the 5' start site, while also protecting the mRNA from nucleases.

According to a more specific embodiment, the mRNA molecules of the present invention further comprise a poly(A) tail comprising 20 to 400 bases at the 3' end.

As used herein, the term "poly(A) sequence", "polyadenine sequence" or "poly(A) tail" refers to an adenine-repeating nucleotide sequence located at the 3' end of an RNA molecule that protects the RNA molecule from degradation by enzymes.

In still another aspect of this invention, there is provided a pharmaceutical composition for preventing or treating an inflammatory disease or an autoimmune disease, comprising the antibody or the antigen-binding fragment thereof; or the nucleic acid molecule encoding the same, as an active ingredient.

In still another aspect of this invention, there is provided a method for preventing or treating an inflammatory disease or an autoimmune disease comprising administering to a subject in need thereof a therapeutically effective amount of a pharmaceutical composition comprising the antibody or the antigen-binding fragment thereof; or the nucleic acid molecule encoding the same, as an active ingredient.

As used herein, the term "preventing" refers to inhibiting the occurrence of a disorder or disease in a subject who has never been diagnosed as having the disorder or disease, but is at risk of developing the condition or disease.

As used herein, the term "treating" refers to (a) inhibiting the progress of a disorder, disease or symptom; (b) alleviating the disorder, disease or symptom; or (c) eliminating the disorder, disease or symptom. The compositions of the present invention, when administered to a subject, significantly block IL-4-mediated signal transduction, thereby inhibiting the progression of symptoms caused by inflammatory or autoimmune diseases, or eliminating or alleviating such symptoms. Therefore, the composition of the present invention may itself be a cell therapy composition, or may be applied as a therapeutic adjuvant for the disease by being administered together with other active ingredients. Accordingly, the term "treatment" or "therapeutic agent" in the present specification includes the meaning of "therapeutic aid" or "therapeutic adjuvant".

As used herein, the term "inflammatory diseases" is a collective term for diseases in which an inflammatory response constitutes the primary pathogenesis.

As used herein, the term "autoimmune disease" is a collective term for diseases in which an excessive or undesired immune response constitutes the primary pathogenesis, and more specifically, for diseases that arise when the induction or maintenance of self-tolerance is impaired, resulting in an immune response against self-antigens and consequent damage to the subject's own tissues.

According to a specific embodiment, the inflammatory or autoimmune disease of the present invention is selected from the group consisting of rhinitis, conjunctivitis, periodontitis, otitis media, sore throat, tonsillitis, pneumonia, gastric ulcer, gastritis, Crohn's disease, colitis, hemorrhoids, gout, ankylosing spondylitis, rheumatic fever, rheumatoid arthritis, rheumatoid polymyalgia, lupus, fibromyalgia, psoriatic arthritis, osteoarthritis, periarthritis of the shoulder, tendonitis, tenosynovitis, periarthritis, myositis, polymyositis, dermatomyositis, hepatitis, cystitis, nephritis, Sjogren's syndrome, multiple sclerosis, inflammatory bowel disease, asthma, type 1 diabetes, psoriasis, eczema, dermatosclerosis, vitiligo, peripheral neuritis, uveitis, autoimmune thrombocytopenia, autoimmune myocarditis, atopic dermatitis, primary liver cirrhosis, dry eye, Goodpasture's syndrome, autoimmune meningoencephalitis, Addison's disease, autoimmune parotitis, dystrophic bullous epidermolysis, epididymitis, glomerulonephritis, Graves' disease, celiac disease, Guillain-Barré syndrome, Hashimoto's disease, hemolytic anemia, myasthenia gravis, amyotrophic lateral sclerosis, pemphigus vulgaris, sarcoidosis, spondyloarthropathy, thyroiditis, vasculitis, myxedema, pernicious anemia, antiphospholipid syndrome, and graft-versus-host disease.

According to a specific embodiment, the rhinitis is nasal polyps or sinusitis.

As used herein, the term "nasal polyp" refers to a pathological condition in which a benign edematous grape-like mucosal mass typically originating from the middle meatus protrudes into the nasal cavity.

As used herein, the term "sinusitis" refers to a condition in which the natural ostia of the paranasal sinuses become obstructed, leading to inadequate ventilation and drainage of the sinuses, thereby causing secondary inflammation and progressive accumulation of purulent secretions.

According to a specific embodiment, the sinusitis is Chronic Sinusitis (CRS).

As used herein, the term "chronic sinusitis" refers to a condition in which the symptoms of sinusitis generally persist for approximately three months or longer. However, the term is not limited thereto and includes any case in which the symptoms persist beyond the usual recovery period of the disease.

According to a specific embodiment, the chronic sinusitis is chronic sinusitis with nasal polyp (CRSwNP).

As used herein, the term "CRS with nasal polyps (CRSwNP)" refers to a condition in which nasal polyps are present in addition to chronic sinusitis, whereas the absence of nasal polyps is referred to as "CRS withour nasal polyps (CRSsNP)"

According to a specific embodiment, the antibody or antigen-binding fragment thereof, or the nucleic acid molecule is administered intranasally.

As used herein, the term "nasal administration" refers to administration of a drug via the nasal cavity or the nasal mucosal surface, and such administration may be performed by a non-invasive route.

Advantageous Effects

The features and advantages of the present invention are summarized as follows:

(a) The present invention provides antigen-binding fragments, more specifically nanobodies, that exhibit markedly high binding affinity for IL-4R and effectively block IL-4-mediated signal transduction, while being capable of directly crossing epithelial cells due to their low molecular weight.

(b) Further, the inventors have identified amino acid substitution variants of the nanobody, as well as bivalent forms thereof, that maximize its binding affinity for IL-4R, and confirmed that these variants exhibit superior nasal epithelial cell permeability and remarkable anti-inflammatory efficacy.

(c) Accordingly, the present invention may be useful as a composition for the treatment of chronic inflammatory diseases, particularly in the form of a nasal spray formulation, while avoiding the various adverse effects associated with systemic administration.

**Brief Description of Drawings**

FIG. 1 shows the final screening process for the anti-IL-4R nanobodies. The y-axis represents the fold-change in OD values between IL-4R and MBP, which reflects the IL-4R specificity of each nanobody clone. Clones highlighted in red indicate the final selected nanobodies with fold-change values exceeding the threshold (1.7).

FIG. 2 shows the results of examining the binding affinity to the IL-4R molecule for the four final nanobody candidates, in which each nanobody was purified and nanobodies at the same concentration were analyzed by ELISA. It was confirmed that the H5 nanobody showed the highest binding affinity to the IL-4R molecule among the four candidates at the same concentration.

FIG. 3 illustrates the results of selecting three nanobody candidates based on the ELISA results to investigate whether binding affinity is related to IL-4 signal-blocking function, and comparing the degree of signal blocking of each nanobody to that of dupilumab at three different concentrations. Reporter cells were incubated with 83 pM IL-4 and each nanobody (or antibody) for 24 hours, and the results showed that dupilumab and the H5 nanobody exhibited the most effective IL-4 signal-blocking effects among the candidates.

FIG. 4 illustrates the results of comparing the signal-blocking effect of the H5 nanobody, which was selected as the final IL-4R-blocking nanobody, with that of dupilumab at three different concentrations.

FIG. 5 illustrates the binding affinity of dupilumab and the H5 nanobody to HEK293 cells as evaluated using a plate reader.

FIGS. 6a to 6d illustrate the modulation of FOXJ1 expression by IL-4, dupilumab, and the nanobody, as determined by immunofluorescence staining and image analysis.

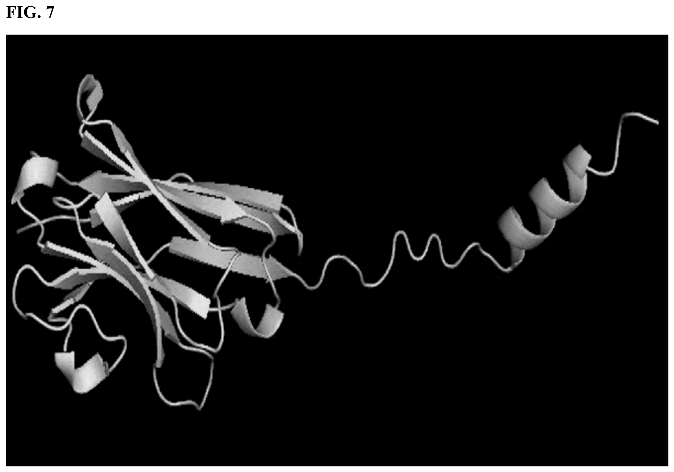

FIG. 7 illustrates the structure of H5 of the present invention as predicted using the AlphaFold 2 program.

FIG. 8 illustrates the results of analyzing the receptor-binding affinities of H5 and its substitution mutants, H5 A110Y and H5 E104R, using surface plasmon resonance (SPR).

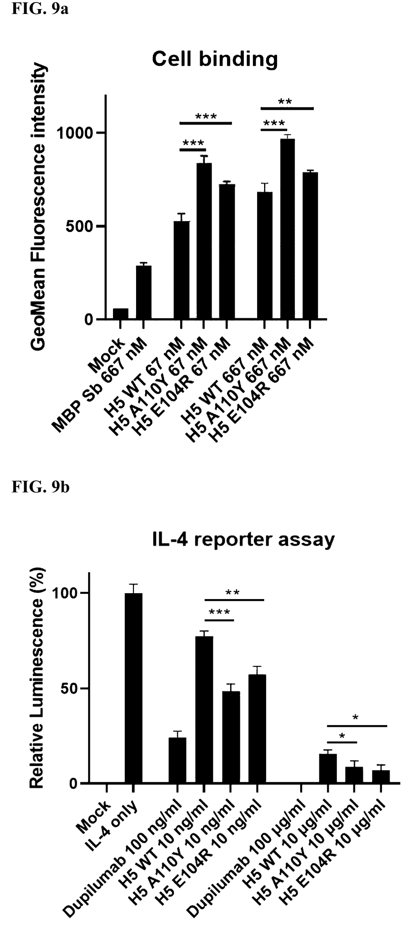

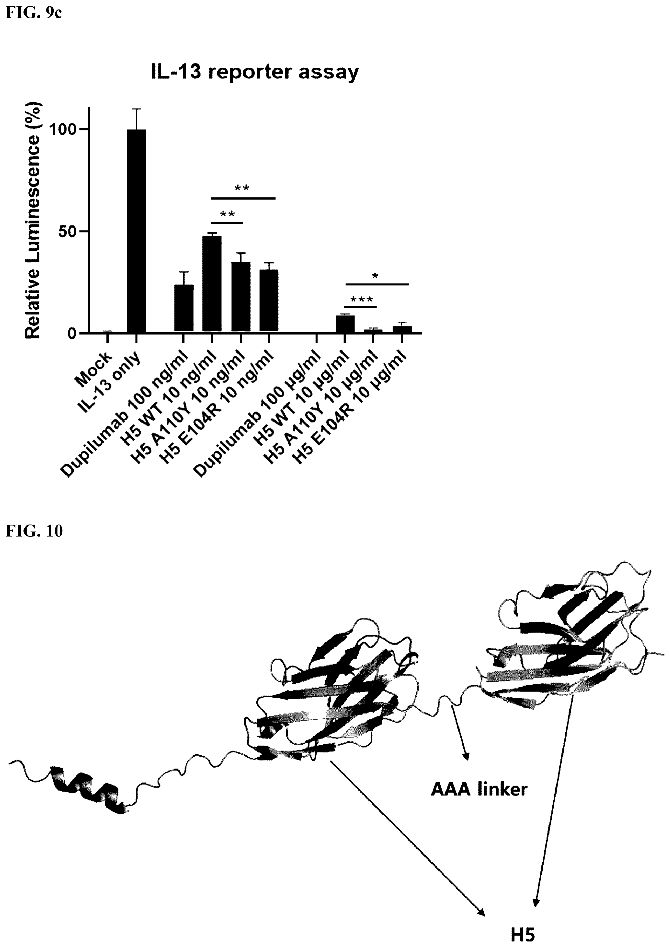

FIG. 9a illustrates the results of analyzing the cellular binding of H5 and its substitution mutants H5 A110Y and H5 E104R. FIG. 9b illustrates the results of a reporter assay analyzing the binding of H5 and its substitution mutants H5 A110Y and H5 E104R to the IL-4 receptor. FIG. 9c illustrates the results of a reporter assay analyzing the binding of H5 and its substitution mutants H5 A110Y and H5 E104R to the IL-3 receptor.

FIG. 10 illustrates the structure of the bivalent nanobody (a dimeric nanobody) of the present invention, as predicted using the AlphaFold 2 program.

FIG. 11 illustrates the results of analyzing the receptor-binding affinities of the H5 nanobody, the bivalent nanobody composed of two H5 wild-type nanobodies, the bivalent nanobody composed of two H5 A110Y substitution mutants, and the bivalent nanobody composed of two H5 E104R substitution mutants, by surface plasmon resonance (SPR).

FIG. 12a illustrates the results of the cellular binding assay of H5, its substitution mutants H5 A110Y and H5 E104R, and the bivalent nanobody of the present invention. FIG. 12b illustrates the results of the reporter assay analyzing the IL-4 receptor-blocking ability of H5, its substitution mutants H5 A110Y and H5 E104R, and the bivalent nanobody of the present invention. FIG. 12c illustrates the results of the reporter assay analyzing the IL-13 receptor-blocking ability of H5, its substitution mutants H5 A110Y and H5 E104R, and the bivalent nanobody of the present invention.

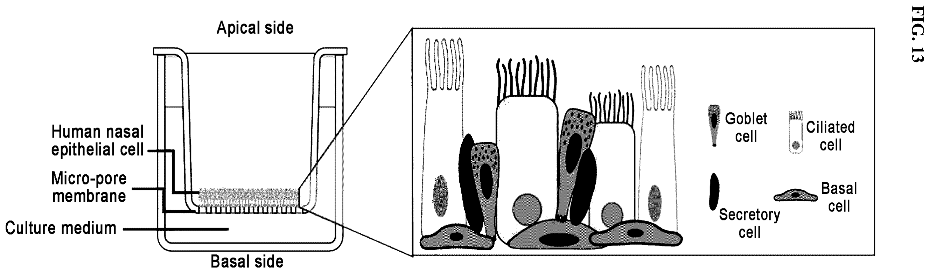

FIG. 13 illustrates the air-liquid interface (ALI) culture method for determining the extent of nasal epithelial cell penetration of nanobodies of the present invention and their effectiveness in preventing or treating inflammation.

FIGS. 14a to 14b illustrate the results of analyzing the mRNA levels of IL-4/IL-13 inflammatory signaling markers (MUC5AC, CCL26, and FOXJ1) by qPCR after different concentrations of the H5 nanobody were added to the air-liquid interface (ALI) culture.

FIG. 15 illustrates the results of analyzing the expression level of the IL-4/IL-13 inflammatory signaling marker MUC5AC by immunostaining after different concentrations of the H5 nanobody were added to the air-liquid interface (ALI) culture.

FIG. 16 illustrates the results of analyzing the lower cell culture medium by Western blotting (WB) after treatment of the apical side of the nasal epithelium in the air-liquid interface (ALI) culture with the substance.

FIG. 17 illustrates the results of analyzing the paracellular permeability of H5, a bivalent nanobody composed of two H5, and dupilumab.

FIG. 18 illustrates the results of analyzing the mRNA expression levels of the tight junction proteins Zo-1 and Occludin in nasal epithelium treated with IL-4/IL-13.

## Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

## Example

## Experimental Methods and Analysis

*Cells*

HEK293T and HEK293 cell lines were purchased from the Korean Cell Line Bank. These cells were cultured in DMEM high glucose medium (Gibco, 11995-065) supplemented with 10% FBS (Gibco, 26140-079) and penicillin/streptomycin (Gibco, 15140-122) at 37°C, 5% $CO_2$.

*Sequence of primers used*

The primers information was referenced from the publication on synthetic nanobody generation (Zimmermann et al., 2018)[1].

FW_c_for: CAA GTC CAG CTG GTG GAA TCG (SEQ ID NO: 10)
FW_c_rev: GCC GCT AGC CGC ACA G (SEQ ID NO: 11)
Link2_c_for: ATA TAT GAA GAC CTC TGC GCG GC (SEQ ID NO: 12)
Link2_c_rev: ATG CAT GGT CTC AGC AGT AAT ACA AAG CAG TAT CTT CCG G (SEQ ID NO: 13)
CDR3_c: GAA GAC CTC TGC GCG GCA GCC 111 111 GGC 111 111 111 111 CCG CTG 111 111 111 111 111 TAT 222 TAC TGG GGT CAG GGC ACC CAA GTT ACC GTT TCT (SEQ ID NO: 14)
5'_flank_for: CGA AAT TAA TAC GAC TCA CTA TAG GGA GAC (SEQ ID NO: 15)
5'_flank_rev: TAT AGC TCT TCA ACT ACC CAT GGA TAT ATC TCC (SEQ ID NO: 16)
3'_flank_for: TAT AGC TCT TCT GCA AGC TTT ATT ATG GCC TC (SEQ ID NO: 17)
tolAK_rev: CCG CAC ACC AGT AAG GTG TGC GGT TTC AGT TGC CGC TTT CTT TCT (SEQ ID NO: 18)
RT primer: CTT CAG TTG CCG CTT TCT TTC TTG (SEQ ID NO: 19)
Long_FX_for: ATA TGC TCT TCT AGT CAA GTC CAG CTG GTG GAA TCG (SEQ ID NO: 20)
Long_FX_rev: TAT AGC TCT TCA TGC AGA AAC GGT AAC TTG GGT GCC C (SEQ ID NO: 21)
qPCR_RD_5'_for: GGG AGA CCA CAA CGG TTT CCC (SEQ ID NO: 22)
qPCR_RD_L_5_rev: GCC GCT AGC CGC ACA GCT C (SEQ ID NO: 23)

*Build a Randomized Convex Scaffold DNA Library*

The pRDV (Addgene, 132696) containing Sb-convex was selected as the template for PCR. The FW_c_for and Link2_c_rev primers were used to amplify the former region of the convex scaffold comprising the CDR1 and CDR2 regions. The Link2_c_for, FW_c_rev, and CDR3_c primers were used to amplify the latter region of the convex scaffold for randomization of the CDR3 region. The randomized primer CDR3_c comprises 10 different trinucleotides, nine of which (111) are enriched in A, S, T, N, and Y (10.6% each), and D, E, Q, R, K, H, and W (5% each), and are characterized by a scarcity of the nonpolar amino acids F, M, V, I, L, and G (2% each)[1]. Another trinucleotide residue(222) lacks amino acids D and A, because these two amino acids are underrepresented at the terminus of the β-sheet[2]. BbsI restriction sites were present at both ends of the former (scaffold) and latter (randomized) convex fragments, which were restricted and ligated to form a convex library with a diversity of $3.17 \times 10^{12}$.

*In-Vitro Transcription*

Both the 5' and 3' regions of the CDR3-randomized convex library were flanked by specific sequences prior to transcription. The 5' flanking region and 3' flanking region were amplified from plasmid pRDV5 comprising Sb-convex (Addgene, 132696) using primers 5'_flank_for and 5'_flank_rev; and 3'_flank_for and tolAK_rev, respectively.

*Ribosome Display*

Ribosomal display has the advantage of enabling the display of approximately $10^{12}$ distinct library members with minimal effort[1]. However, this method has not been widely applied due to several reasons related to reagents and unfavorable RNase activity[3]. To overcome the technical obstacles, we adopted an in vitro translation kit, PUREfrex2.1 (GeneFrontier, PF213-0.25-EX) [1]. The kit lacks glutathione oxidase (GSSG) and disulfide bridge isomerase (DsbC), which were further supported to form disulfide bond folding. A 70 ng RNA library (approximately $1.7 \times 10^{12}$ molecules) was used as input, and the experimental procedure followed the manual. For ribosome complex formation, the in vitro translation reaction was carried out at 37°C for 1 hour. Prior to ribosome display, 12 μL of Dynabeads™ MyOne™ Streptavidin T1 beads (Invitrogen, 65601) were washed twice with 0.5% WTB-BSA buffer (50 mM Tris/acetate, pH 7.4; 150 mM NaCl; 50 mM MgAc$_2$; supplemented with BSA). The magnetic beads were then blocked with WTB-BSA buffer for at least 20 minutes. The ribosomal complex was added to 100 ul of panning solution (WTB-D-BSA (WTB-BSA supplemented with 0.1% tween20) supplemented with 500 ug of heparin and 1 ul of RNaseIn (Promega N2611)), and the mixture was centrifuged at 20,000 x g for 5 minutes. The supernatant was mixed with biotinylated IL-4R (Acro Biosystems, ILR-H82E9) and incubated for 2 hours on ice. The magnetic beads were washed three times with WTB-B-BSA and the panning-IL-4R mixture was added to the beads and incubated for 1 hour on ice. The whole mixture was then washed three times with WTB-D (50 mM Tris/acetate, pH 7.4, 150 mM NaCl, 50 mM $MgAc_2$, supplemented with 0.1% Tween20). The supernatant was purified using a RNeasy Kit (Qiagen, 74004) with an elution volume of 15 μL.

*Reverse transcription*

RNA eluted from the ribosomal display was reverse transcribed using SuperiorScript III reverse transcriptase (Enzynomics, RT006M) in a total volume of 30 ul according to the manual. The resulting cDNA was purified using a PCR purification mini-kit (Favorgen, FAGCK 001-1) in a 30ul elution volume. 1 ul of the eluate was used as a template for qPCR analysis and 29 ul of the elution was used for amplification. The resulting DNA purification in the form of cDNA was amplified by PCR using Long_FX_for and Long_FX_rev primers. The total reaction volume was 100 ul and was divided into two tubes at the end of the reaction.

*qPCR*

The qPCR assay was used to assess the quality of cDNA resulting from ribosomal display and phage display selection, and to monitor enrichment of the library during the selection step[4]. For the experiments, a QuantStudio 3 Real-time PCR instrument (Applied Biosystems) was used with AccuPower® 2X Greenstar qPCR master mix (Bioneer, K-6251). The PCR program conditions were as follows: 95°C, 2 minutes (initial denaturation) / 95°C, 10 seconds; 63°C, 30 seconds (denaturation, annealing, extension, measurement) / Melting curve steps followed the default settings in the machine manual.

*Electroporation*

The enriched nanobody library was introduced into the phagemid vector pDXinit (Addgene, 110101) using FX cloning[5]. 350 ul of E. coli SS320 (Lucigen, 60512-1) was thawed on ice, and 50 ul of the phagemid's ligation mix comprising the enriched library was added to an electroporation cuvette (Biorad, 1652086) containing SS320 and gently mixed by pipetting. The cell mixture was pulsed with a Biorad Gene Pulser II electroporation system using 2.4kV, 25uF, and 300Ω. The electroporated cells were immediately transferred to 25 ml of SOC medium and incubated 37°C, 160 rpm for 30 minutes. The harvested cultures were then transferred to 225 ml of 2TY medium supplemented with 200 ug/ml ampicillin and 2% glucose and incubated overnight at 37°C, 160 rpm.

*Production and purification of phages*

1 ml of electroporation pre-culture was added to 50 ml of 2YT medium (supplemented with 200 ug/ml ampicillin and 2% glucose) and incubated at 37°C , 160 rpm until $OD_{600}$ = 0.6. Then 10 ml of the culture was mixed with 30 ul of M13KO7 helper phage (NEB, N0315S) and incubated at 37°C for 1 hour without shaking. The incubated culture was centrifuged at 5,000 x g for 10 minutes and the supernatant was removed. The pellet was resuspended in 50 ml of 2YT medium (supplemented with 200 ug/ml ampicillin and 25 ug/ml kanamycin) and incubated overnight at 37°C and 160 rpm.
The overnight incubated cultures were centrifuged at 6,000 x g, 4°C for 30 minutes. Transfer 40 ml of the supernatant to a new 50 ml tube, add 10 ml of PEG6000/NaCl, and invert the tube approximately 5 times. The mixture was incubated overnight on ice, then centrifuged at 10,000xg, 4°C for 1 hour. After removing the supernatant, the tubes were gently washed with 40 ml of PBS, followed by resuspension in 1 ml of PBS. The resuspended phage was centrifuged at 20,000 x g, 4°C for 5 minutes and the supernatant was collected in a new tube. The titers of the collected phages were measured using UV-visible spectroscopy at 269 nm and 320 nm and then calculated by the following equation:

$$\frac{phages}{ml} = \frac{(A269 - A320) \times 6 \times 10^{16}}{1900}$$

*Phage Display*

E. coli SS320 was cultured in 50 ml of 2YT medium (supplemented with 10 ug/ml tetracycline) and half of a 96-well plate was coated with 100 ul of 67 nM neutravidin from 4°C one day prior to phage display.

- The first round of phage displays

Neutravidin-coated plates were washed with 250 ul of TBS for each well and blocked with 250 ul of TBS-BSA (TBS supplemented with 0.5% BSA). Biotinylated IL-4R was added to 4.9 ml of purified phages ($10^{12}$ phages/ml) to a final protein concentration of 50 nM, and incubated for 2 hours on ice. Neutravidin-coated plates were washed with 250ul TBS-BSA-D (TBS supplemented with 0.5% BSA and 0.1% Tween 20). 100 ul of the phage-IL-4R mixture was added to each well of the plate and incubated for 1 hour on ice. Each well of the plate was washed three times with 150 ul of TBS-D (TBS supplemented with 0.1% Tween 20), and the plate was dried on paper towels for 2 minutes after each wash step. 100 ul of PD elution buffer (TBS with 0.25 ml/ml trypsin added as powder) was added to each well, and the plate was incubated for 10 minutes at room temperature. The eluted solution was transferred to a new tube, and 40 ul of AEBSF solution (Merck, A8456) was added to the tube. 1 ul of the sample was analyzed by qPCR, and the remaining sample was added to 50 ml of cultured SS320. The mixture was incubated at 37°C for 1.5 hours without shaking, then 50 ml of the mixture was transferred to 200 ml of 2YT medium (supplemented with 200 ug/ml ampicillin and 2% glucose) and incubated at 37°C, 160 rpm overnight.

- The second round of the phage display

Phages generated from the first phage display were harvested and purified in TBS-BSA-D($5\times1012$ phages/ml). Biotinylated IL-4R was added to 100ul of phage solution at a concentration of 50nM and incubated for 2 hours on ice. Dynabeads™ MyOne™ StreptavidinC1 beads (Invitrogen, 65001) 12 ul were washed twice with 500 ul of TBS-WTB-BSA, then blocked with 500 ul of WTB-BSA on ice for at least 20 minutes. The magnetic beads were washed three times with 500 ul of TBS-BSA-D, resuspended, and incubated with the phage-IL-4R complex solution for 1 hour on ice. After incubation, the mixture was washed with 500 ul of TBS-BSA-D, resuspended in 100 ul of 'competition buffer', which is TBS-BSA-D supplemented with 5 uM of non-biotinylated IL-4R (Sino Biological, 10402-H08H), and incubated for 3 min on ice. Competitive non-biotinylated IL-4R was washed away twice with 500 ul of TBS-D. The beads were resuspended in 100 ul of PD elution buffer and incubated for 10 minutes at room temperature. To the resulting solution, 0.8 ul of ABESF was added and pipetted to mix, 1 ul of the solution was used for qPCR analysis, and the remaining solution was used for transfection of SS320. E. coli SS320 was cultured in 2YT medium supplemented with 200 ug/ml ampicillin and 2% glucose 37°C, 160 rpm overnight

*Production and Purification of Biotinylated MBP*

Plasmid pBXNH3CA_MBP (addgene, 132700) was transformed into E.coli BL21 for expression. This was cultured in TB medium supplemented with 100 ug/ml ampicillin for 4 hours at 37°C, 160 rpm. For expression and biotinylation, arabinose and biotin were added at concentrations of 0.02% and 100uM, respectively. The cultures were then incubated overnight at 22°C, 160 rpm for expression. Overnight cultures were sonicated and purified using MBP MiniExcellose® (Takara, AEx-MC-M03).

*Enzyme-linked immunosorbent assay (ELISA)*

Using the FX cloning method, the enriched library was cloned into the pSBinit vector (addgene, 110100) in two rounds by phage display[5]. The cloned plasmids were transformed into E. coli SS320 by electroporation, then incubated overnight at 37°C, 160 rpm, and the plasmids were extracted. The extracted plasmid was transformed into E. coli BL21 (Enzynomics, CP110) and plated on LB-agar plates supplemented with 25 ug/ml chloramphenicol and incubated overnight at 37°C.

1.2 ml of TB medium supplemented with 25 ug/ml chloramphenicol was prepared in each well of a 96-well deep-well plate labeled 'pre-culture'. From the incubated plate, 95 colonies were selected and each colony was inoculated into each well and the first well was filled with the positive control pSb_init comprising the MBP nanobody Sb_MBP#1 (addgene, 132699). Deep well plates were sealed with gas permeability and grown 37°C, 300 rpm for 4 hours. A new 96-well deep-well plate filled with 1 ml of pre-warmed TB medium supplemented with 25 ug/ml chloramphenicol was labeled 'expression culture'. 50ul of 'pre-culture' was added to the corresponding wells of the expression culture and the plate was incubated at 37°C, 300 rpm for 2 hours and 22°C, 300 rpm until the OD600 reached 0.4 - 0.8. After sufficient growth, 0.02% (wt/vol) of L-(+)-arabinose was added to induce expression, and the plates were incubated overnight at 22°C, 150 rpm. The next day, cells were collected by centrifugation at 5,000 x g, 4°C for 15 min. Pallets of 'pre-cultures' were stored at -20°C for DNA purification, and pallets of 'expression cultures' were resuspended with 100 ul of periplasmic extraction buffer (20% sucrose, 50 mM Tris pH8.0, 0.5 mM EDTA, and 0.5 ug/ml of lysozyme (labeled DW)) by intensive vortexing.

After incubation for 30 minutes on ice, 900 ul of TBS supplemented with 1 mM $MgCl_2$ was added to each well. The plates were centrifuged at 5,000 x g, 4°C for 15 minutes and the supernatant was used as periplasmic extract for ELISA. Two 96-well immunoplates were coated with 100 ul of 5 ug/ml protein A solution and incubated overnight at 4°C with adhesive seals. The plates were washed with 250 ul of TBS per well and then blocked with 150 ul of TBS-BSA per well. Next, 100 ul of anti-c-Myc antibody (Biolegend, 626802) diluted 1:2,000 in TBS-BSA-D was added per well and incubated for 20 minutes. After washing three times with 250 ul of TBS-D, 80 ul of TBS-BSA-D was added to each well to compare nanobody binding to IL-4R, and 20 ul of the same cytoplasmic extract was added in parallel to compare the binding of the nanobody to IL-4R and MBP, followed by incubation for 20 minutes. The plate was washed with 250 ul of TBS-D per well, 100 ul of 50nM MBP was added to the first two wells, 100 ul of 50 nM biotinylated IL-4R was added to each well, and the plate was incubated for 20 minutes. After washing three times with 250 ul TBS-D, 100 ul of 1:5,000 streptavidin-peroxidase (Invitrogen, 434323) diluted in TBS-BSA-D was added to each well and incubated for 20 minutes. The plate was washed three times again with TBS-D, and 100 ul of TMB substrate (biolegend, 421101) was added to each well. The reaction took about 15 minutes until the individual wells turned blue. The absorbance was measured at 650 nm with a plate reader.

*Production and Purification of nanobodies*

The nanobody clone in the expression vector (pSBinit) was transformed into E. coli BL21 and incubated overnight at 37°C, 160 rpm. 2 ml of the overnight cultures were inoculated into 200 ml of TB medium supplemented with 25 ug/ml chloramphenicol and incubated at 37°C, 200 rpm until $OD_{600}$ = 0.6, then the cultures were transferred to 22°C, 150 rpm for 1 hour. To induce expression, 0.02% arabinose was added and incubated overnight at 37°C, 150 rpm. The overnight cultures were centrifuged at 5,000xg, 4°C for 15 min, and the pallet was resuspended in 20 ml of ambient cytoplasmic extraction buffer on ice for 30 minutes. After incubation, 180 ml of TBS supplemented with 1 mM $MgCl_2$ was added and centrifuged at 5,000 x g, 4°C for 15 minutes.

1 ml of TALON® Superflow™ (Cytiva, 28957502) slurry was pre-equilibrated with TBS pH 8.0. After binding, the beads were washed three times with wash buffer (50 mM Tris pH 8.0, 300 mM NaCl, 5 mM imidazole) and the nanobodies were eluted with elution buffer (50 nM Tris pH 8.0, 300 mM NaCl, 200 mM imidazole). The eluted nanobodies were buffer exchanged overnight using a Slide-A-Lyzer® Dialysis Cassette (Thermo, 66330).

*STAT6-Luciferase Reporter System for Measuring IL-4R Inhibition*

The HEK293 cell line was stably transfected with human STAT6 (addgene, 81950) by a puromycin-resistant lentiviral system and pSTAT6-induced luciferase (addgene, 35554) by a blasticidin-resistant system to allow stable expression of luciferase under the control of the STAT6 response to IL-4 signaling[6,7]. 5 x $10^4$ reporter cells were seeded into each well of a 96-well cell culture plate and incubated with either dupilumab or nanobodies in hIL-4 and serum-free RMPI medium for 24 hours under 37°C and 5% $CO_2$ environment. Cell lysates were transferred to white 96-well plates and 50 ul of luciferase substrate (Promega, E1501) was added. Random luminescence units were measured with a SpectraMax® M5 luminometer (Molecular Devices).

*Immunofluorescence staining and image analysis*

HNE cells in transwells were washed with PBS and fixed with 500 ul of 4% paraformaldehyde (Biosesang, P2031) for 30 minutes at room temperature. HNE cells were then incubated with 100 ul (1:200) of FOXJ1 primary antibody (GeneTex, GTX114408) for 1 hour, followed by incubation with 100 ul (1:1000) of mouse anti-rabbit FITC for 30 minutes and washed three times with PBS. Cells were photographed on a Zeiss LSM 700 using the parameter settings of the 405 and 488 nm lasers and the Z-stack multidimensional acquisition function was applied. Images of Z-stack slices were obtained with a spacing of 8um.

*In-silico affinity maturation*

After predicting the structure of the nanobody through AI-based structure prediction, we identified the binding structure with the receptor. Based on the identified receptor binding structure and computer program-based binding enhancement techniques, we identified substitutional mutations H5 A110Y and H5 E104R that can increase binding to the receptor. The affinity of the discovered mutant H5 was analyzed by surface plasmon resonance (SPR).

*Cell Surface Antigen Binding & Reporter Cell Assay*

The cell binding capacity of H5 nanobodies and mutant H5 was analyzed by cell surface antigen binding assay, and the IL-4 and IL-13 receptor blocking capacity was analyzed by IL-4 and IL-13 reporter assay, respectively.

The experiments to evaluate the receptor blocking ability were performed as follows: A reporter cell line was constructed by inserting the STAT6 gene and a luciferase gene that can be expressed by phosphorylated-STAT6 into HEK293 cells using lentivirus. 5 x $10^4$ reporter cells were treated with IL-4/IL-13 and nanobodies in 96-well plates and incubated in 37°C, 5% $CO_2$ for 24 hours. After removing all culture medium, 20 $\mu$l of lysis buffer (25 mM Tris pH 8.0, 4 mM EGTA, 10% glycerol, and 10 $\mu$l of Triton X-100 in 1 ml of DW) was treated, and luminescence was measured by adding 50 $\mu$l of luciferin substrate.

*Bivalent Nanobody*

To maximize the receptor blocking ability of the nanobodies of the present invention, bivalent nanobodies (Bivalent Nanobodies) in the form of dimers were prepared by connecting H5 or its substituted mutant nanobodies with peptide linkers. The receptor affinity of the prepared bivalent nanobodies was analyzed by surface plasmon resonance (SPR). In addition, the cell binding capacity of the prepared bivalent nanobodies was analyzed by cell surface antigen binding assay, and the IL-4 and IL-13 receptor blocking capacity was analyzed by IL-4 and IL-13 reporter assay, respectively.

The AAA peptide linker is used herein as an example implementation.
AAA (SEQ ID NO: 9)

*Air-Liquid Interface Culture*

To evaluate the nasal epithelial cell penetration efficiency and the effects of preventing or treating inflammation of the nanobody or bivalent nanobody developed in the present invention, an air-liquid interface (ALI) culture was prepared. The air-liquid interface culture was prepared by culturing human nasal epithelial cells on top of a micro-pore membrane, which is a permeable membrane, and placing an air layer on the top of the nasal cells and a liquid culture medium on the bottom of the nasal cells.

Air-liquid interface cultures were performed as follows: Human nasal epithelial cells were obtained from rhinitis/nasal tumor patients, and primary cultured nasal epithelial cells in secondary passage were cultured using transwells in culture medium comprising basal epithelial growth medium. After 3 days of submerged cell culture, the top portion of the culture medium was removed to perform air-liquid interface (ALI) culture. Two weeks after ALI culture, fully differentiated nasal epithelial cells were used for experiments.

Air-Liquid Interface Culture was used to determine the receptor blocking ability of H5 and its effectiveness in treating inflammation under ex-vivo conditions. Markers for inflammation were MUC5AC, CC126, and FOXJ1.

Furthermore, in an Air-Liquid Interface Culture, nasal epithelial cells were treated on top of the material and Western blotting was performed on the cell culture at the bottom to determine cell permeability.

*Analysis of tight junction proteins in nasal epithelial cells*

After treatment of nasal epithelial cells with IL-4/IL-13, we measured the expression of the tight junction proteins Zo-1 and Occuldin to determine changes in cell permeability in response to IL-4/IL-13-induced inflammation.

*Sequence of Nanobody Candidates*

H5 CDR Sequence (SEQ ID NO: 1) : DKGREYTLARAKYWYW
H5 CDR sequence, E5R mutation (SEQ ID NO: 2): DKGRRYTLARAKYWYW
H5 CDR sequence, A11Y mutation (SEQ ID NO: 3): DKGREYTLARYKYWYW
H5 CDR sequence, E5R + A11Y mutation (SEQ ID NO: 4):
DKGRRYTLARYKYWYW
H5 Full-length sequence (SEQ ID NO: 5):

QVQLVESGGGSVQAGGSLRLSCAASGSISSITYLGWFRQAPGKEREGVAALS

TSSGTTYYADSVKGRFTVSLDNAKNTVYLQMNSLKPEDTALYYCAAADKGREYTLA

RAKYWYWGQGTQVTVSAGRAGEQKLISEEDLNSAVDHHHHHH

H5 full-length sequence, E104R mutation (SEQ ID NO: 6):

QVQLVESGGGSVQAGGSLRLSCAASGSISSITYLGWFRQAPGKEREGVAALS

TSSGTTYYADSVKGRFTVSLDNAKNTVYLQMNSLKPEDTALYYCAAADKGR

RYTLARAKYWYWGQGTQVTVSAGRAGEQKLISEEDLNSAVDHHHHHH

*H5 full-length sequence, A110Y mutation (SEQ ID NO: 7):

QVQLVESGGGSVQAGGSLRLSCAASGSISSITYLGWFRQAPGKEREGVAALS

TSSGTTYYADSVKGRFTVSLDNAKNTVYLQMNSLKPEDTALYYCAAADKGREYTLA

RYKYWYWGQGTQVTVSAGRAGEQKLISEEDLNSAVDHHHHHH

H5 full-length sequence, E104R + A110Y mutation (SEQ ID NO: 8):

QVQLVESGGGSVQAGGSLRLSCAASGSISSITYLGWFRQAPGKEREGVAALS

TSSGTTYYADSVKGRFTVSLDNAKNTVYLQMNSLKPEDTALYYCAAADKGRRYTLA

RYKYWYWGQGTQVTVSAGRAGEQKLISEEDLNSAVDHHHHHH

**Experimental results**

*The Final Steps in anti IL-4R Nanobody Screening*

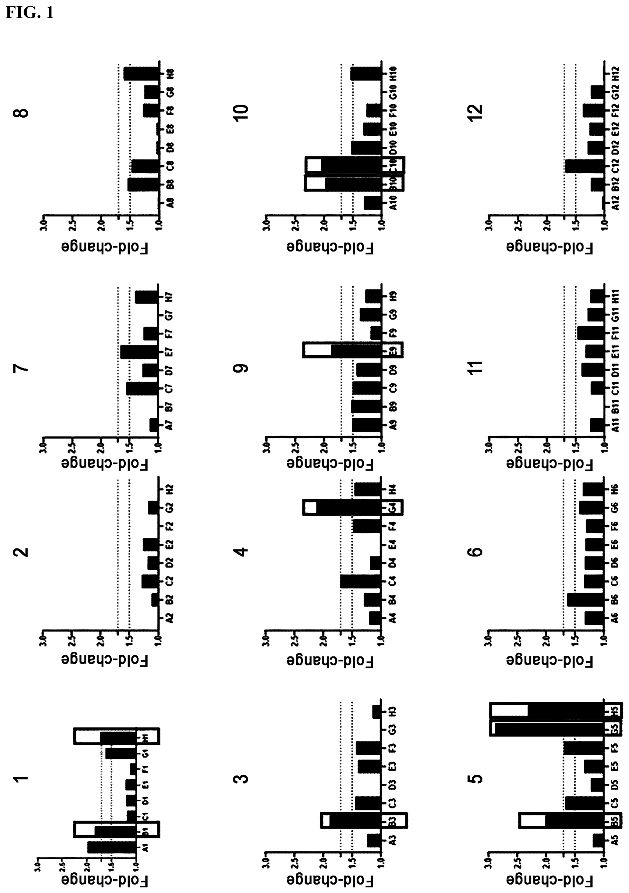

After ribosome display and two phage displays, approximately 102 nanobody clones were screened. To enrich the pool of these nanobodies, ELISAs were performed for IL-4R and MBP. The y-axis of the graph exhibits the fold-change of OD values for IL-4R and MBP, which indicates the specificity of each nanobody clone for IL-4R. The clones labeled with red boxes are the final selected nanobodies with fold-change values higher than the threshold (1.7). Excluding overlapping sequences, the inventors ultimately obtained four different nanobody sequences, H5, G5, E9, and B3 (Figure 1).

*Binding affinity of nanobody candidates for IL-4R*

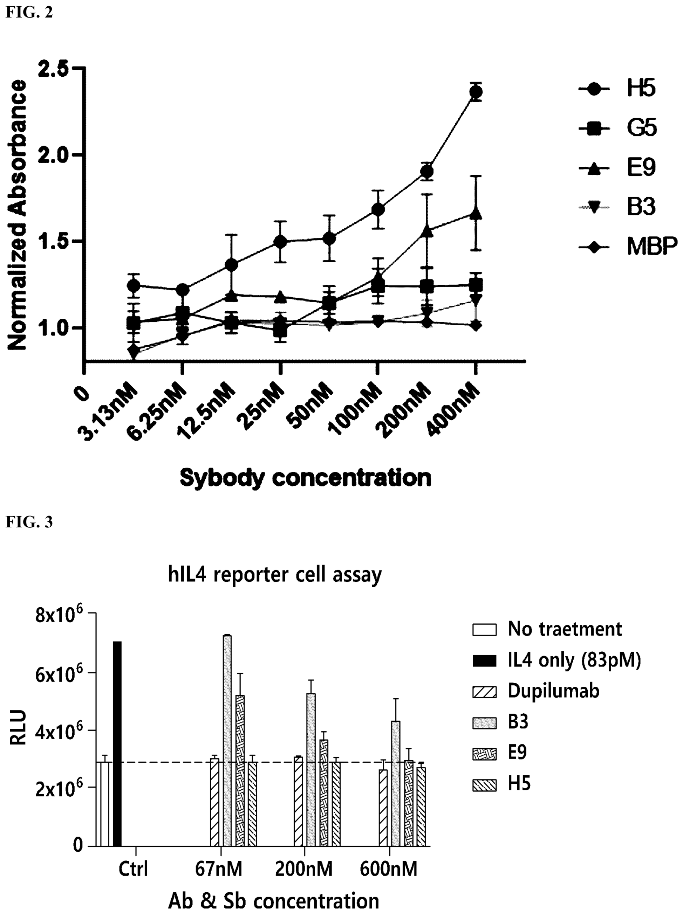

Four final nanobody candidates against IL-4R were selected through ribosome display, phage display, and ELISA. Each nanobody was purified, and an ELISA was performed to determine the binding affinity of nanobodies at the same concentration to the IL-4R molecule. As a result, it was confirmed that the H5 nanobody exhibited the highest binding affinity to the IL-4R molecule among the four candidates at the same concentration (Figure 2).

*IL-4 signaling blocking activity of nanobody candidates*

Three nanobody candidates were selected based on previous ELISA results to investigate whether binding affinity correlates with IL-4 signaling blocking function. The signal blocking ability of each nanobody at three different concentrations was compared to that of dupilumab. Reporter cells were incubated with 83 pM IL-4 and each nanobody (or antibody) for 24 hours. The results showed that dupilumab and H5 nanobodies were the most effective IL-4 signaling blockers among the candidates (Figure 3).

*Comparison of the IL-4 Signaling Blocking Activity of Dupilumab and H5*

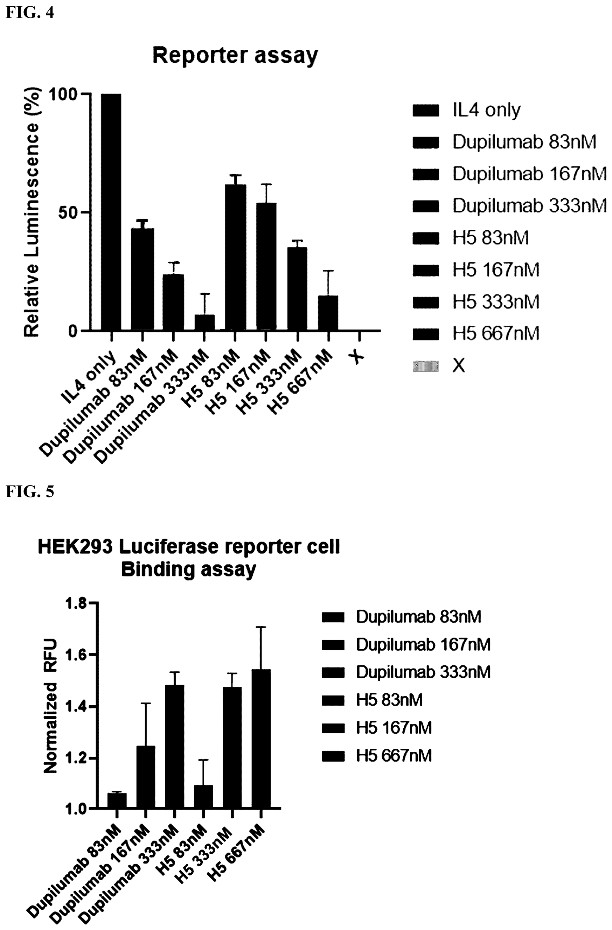

Based on our previous results, we selected the H5 nanobody as the final IL-4R blocking nanobody. The signal blocking activity of each nanobody at three different concentrations was compared to dupilumab. Reporter cells were incubated with 667 pM of IL-4 and the nanobody (or antibody) for 24 hours. We found that H5 nanobodies exhibited similar blocking activity to dupilumab at approximately 2-fold concentration (Figure 4).

*Comparison of cell binding affinities of Dupilumab and H5*

The binding affinity of dupilumab and H5 nanobodies to HEK293 cells was evaluated by plate reader. Anti-human IgG-FITC was used as a secondary antibody for dupilumab, and anti-Myc-FITC was used as a secondary antibody for H5 nanobodies. The fluorescence units of each antibody (nanobody) were normalized to the fluorescence units of each secondary antibody. The results of FIGS. 4 and 5 suggest that the signal blocking activity is dependent on the binding of the antibody (nanobody) to the cell.

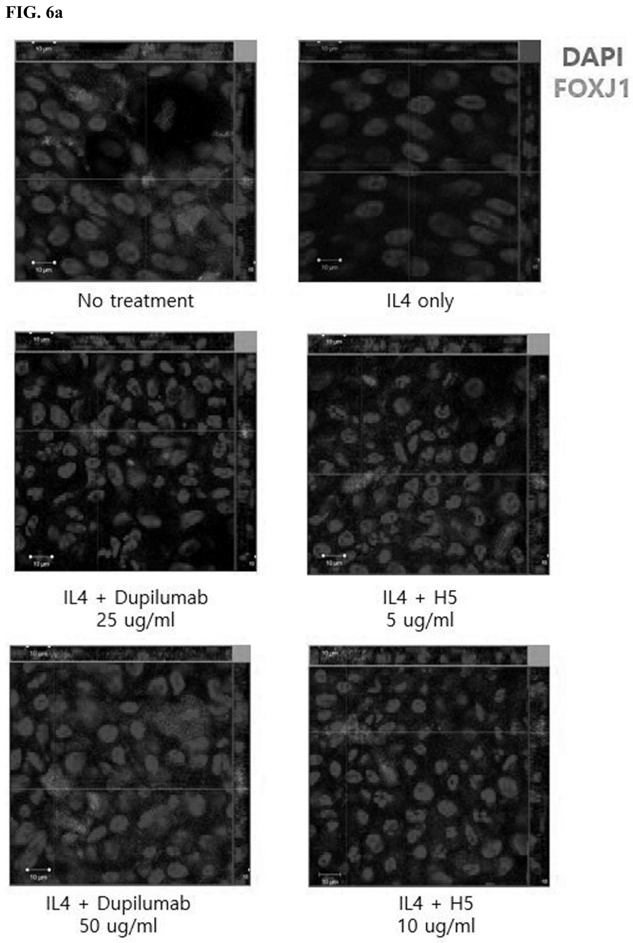

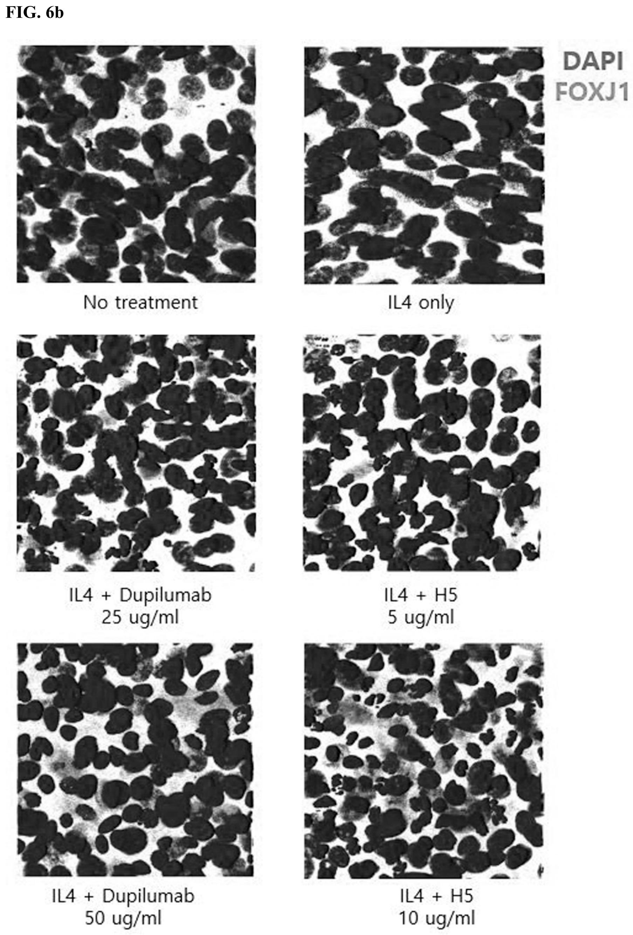

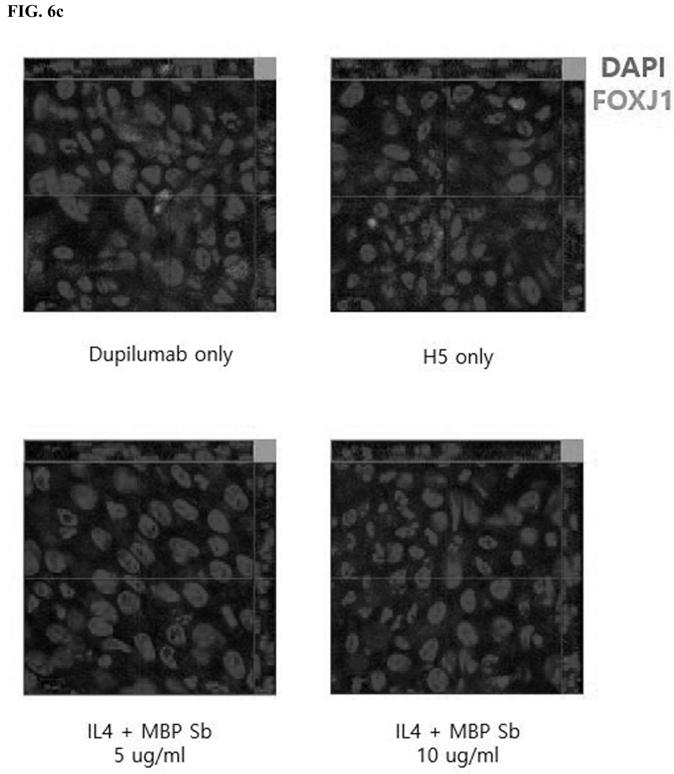

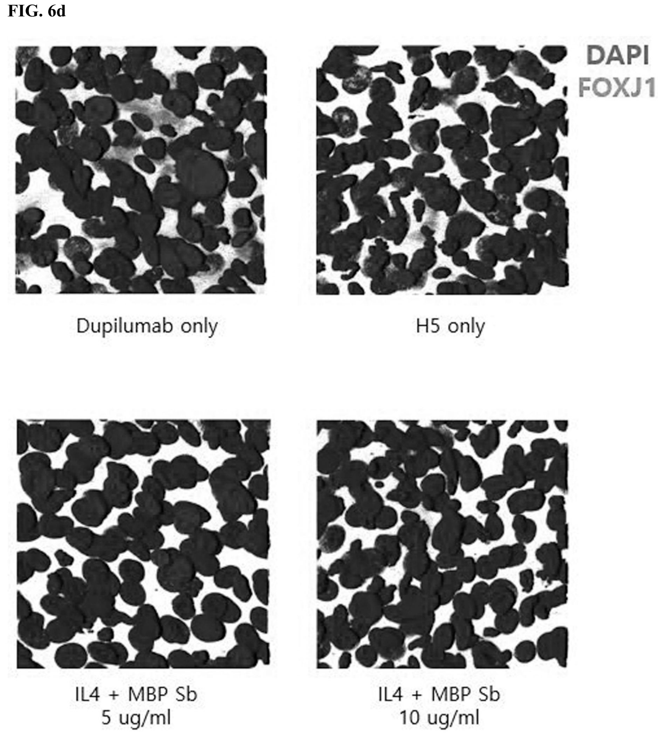

*Regulation of FOXJ1 expression by IL-4, dupilumab, and nanobodies (Figure 6)*

FOXJ1 is a member of the forkhead box family of transcription factors, and its expression is suppressed by IL-4/IL-13 signaling, while STAT6 becomes phosphorylated. Figure 6a is an ortho image of human nasal epithelial cells (HNE) treated with IL-4, dupilumab, and H5 nanobodies. Administration of dupilumab and H5 was shown to block IL-4 signaling and subsequently induce the expression of FOXJ1. FIG. 6c shows that FOXJ1 expression is induced when dupilumab and H5 are treated without IL-4, indicating that the antibodies and nanobodies do not have any agonistic activity against the IL-4R. However, the expression of FOXJ1 is inhibited upon treatment with both IL-4 and anti-MBP nanobodies, suggesting that the nanobody scaffold itself does not have any antagonistic effect on IL-4R. FIGS. 6b and 6c are 3D images of the corresponding HNE samples.

*In-silico affinity maturation was used to maximize the receptor binding capacity of the nanobodies.*

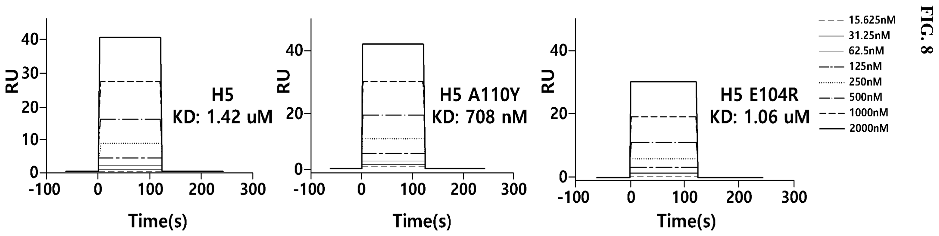

For the H5 A110Y and H5 E104R mutants, which were identified through AI-based structural prediction methods and computer program-based binding affinity enhancement techniques, the KD were measured to be 708 nM and 1.06 nM, respectively. Compared to WT H5 which has a KD of 1.42 uM, up to a 2-fold increase in binding was observed (Figure 8).

*H5 and its substituted mutants and Bivalent Nanobodies are superior to dupilumab in blocking the receptor.*

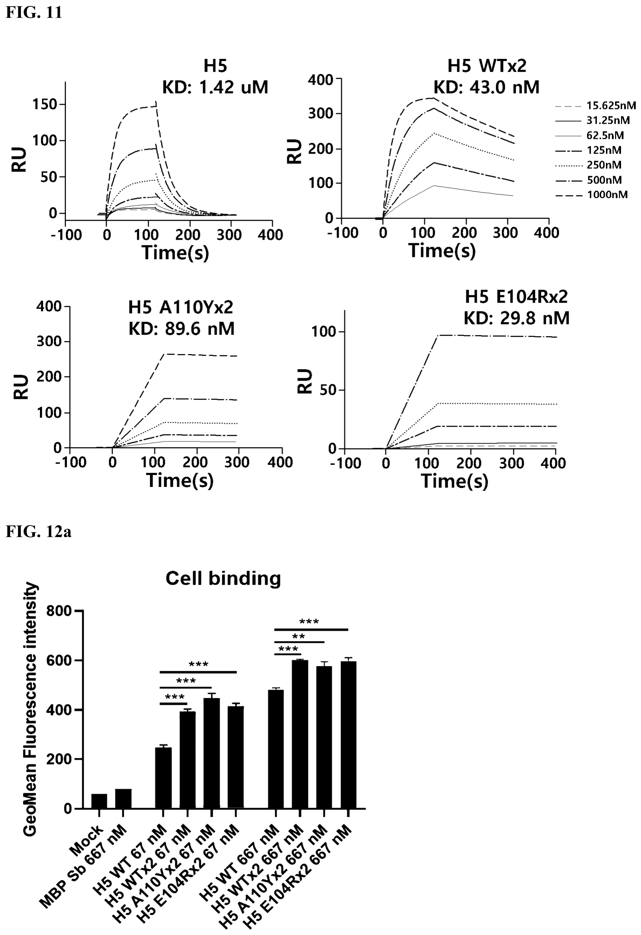

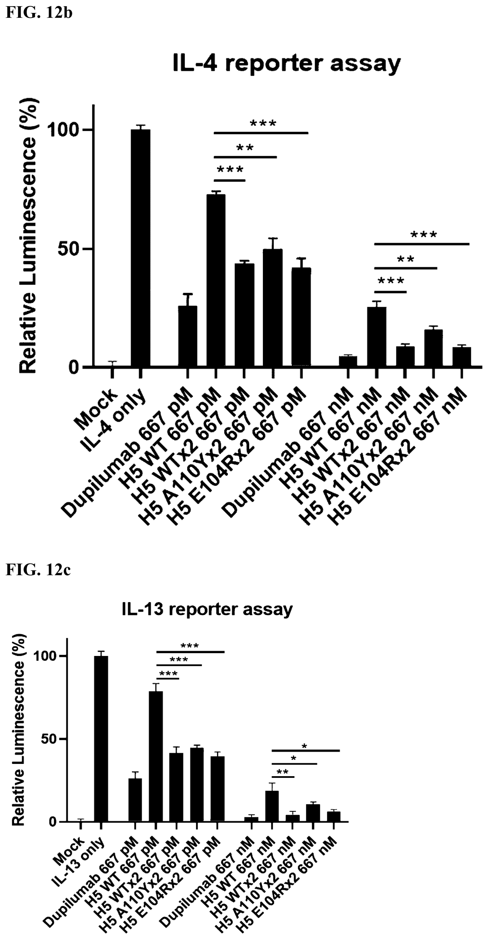

When analyzing the cell binding and receptor blocking capacities of the H5, H5 A110Y mutant and H5 E104R mutant and the prepared bivalent nanobodies of the present invention, it was confirmed that the bivalent nanobodies exhibited up to a 50-fold increase in binding affinity (FIG. 11). Furthermore, the bivalent nanobodies were found to be superior to the single nanobodies (H5, H5 A110Y mutant, and H5 E104R mutant) not only in molecular binding but also in cell binding and receptor blocking ability (Figures 12a to 12c).

*The nanobodies of the present invention were found to be superior to dupilumab in cell permeability and treatment of inflammation.*

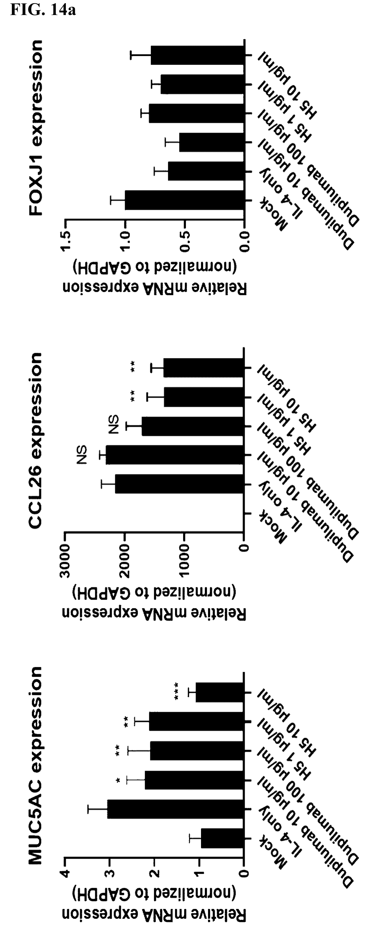

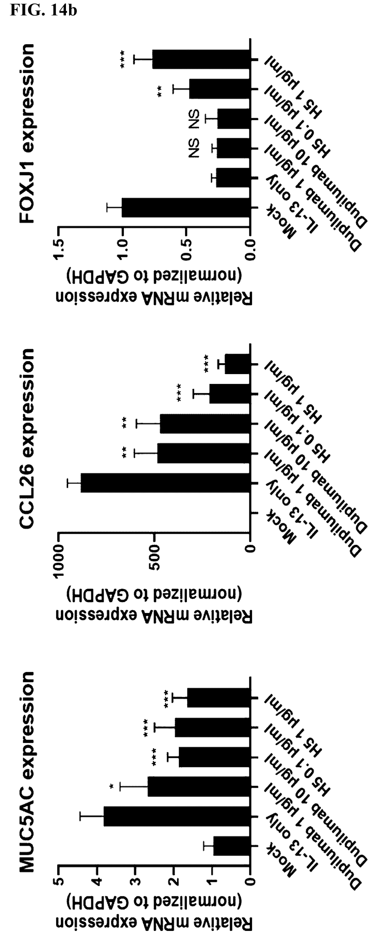

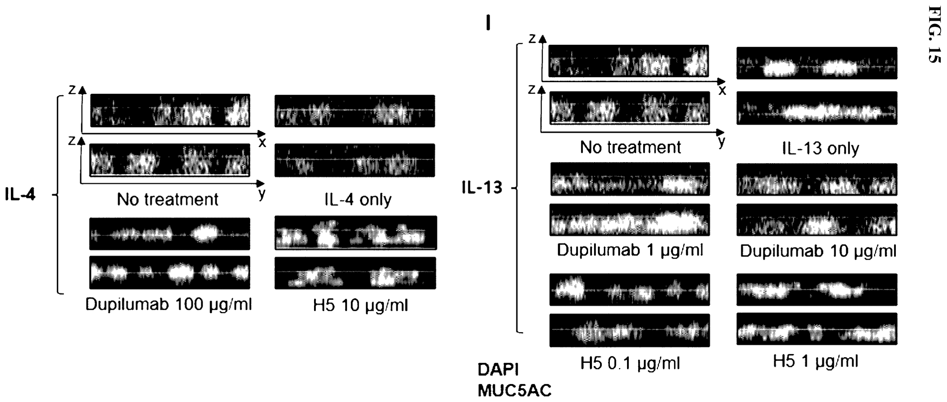

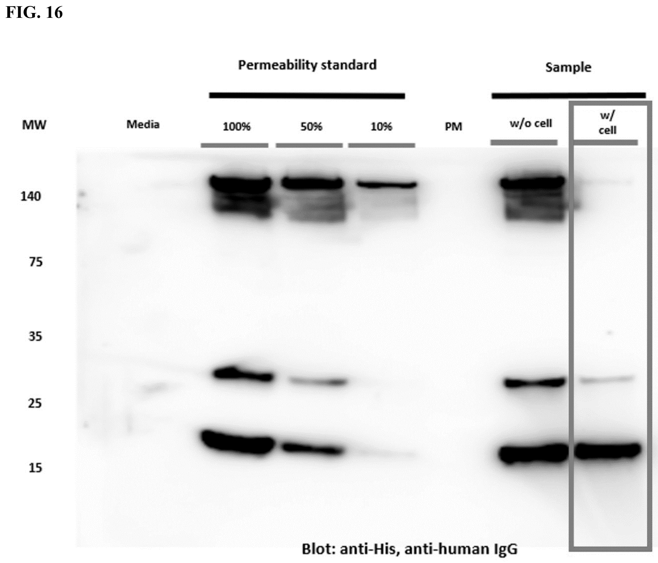

Air-Liquid Interface Culture experiments demonstrated that H5 was superior to dupilumab in nasal cell permeability (Figure 16) and blocking or treating inflammation (Figure 14a, Figure 14b, and Figure 15).

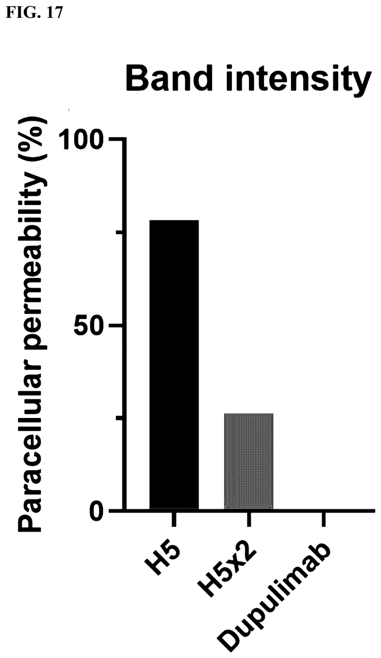

MUC5AC, CCL26, and FOXJ1 were used as markers for inflammation. MUC5AC is a marker that is overexpressed by IL-4/IL-13 signaling, CCL26 is a marker that is overexpressed by IL-4/IL-13 signaling, and FOXJ1 is a marker that is inhibited by IL-4/IL-13 signaling. Furthermore, H5 and H5 bivalent nanobodies showed better paracellular permeability than dupilumab (Figure 17).

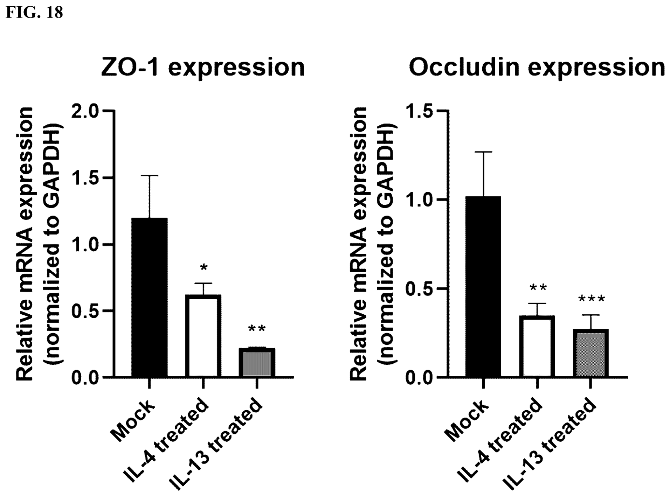

On the other hand, the expression levels of Zo-1 and Occludin, which correspond to tight junction proteins in nasal epithelial cells, were analyzed by measuring their mRNA levels. The expression of both proteins was significantly reduced upon treatment with IL-4 or IL-13, confirming that cell permeability is increased in patients with inflammatory diseases in which IL-4 or IL-13 is overexpressed (FIG. 18). Taking the above results into account, it can be concluded that the nanobodies of the present invention can easily penetrate epithelial cells due to their small molecular weight and that the adhesion of nasal cells in inflammatory patients is reduced, and therefore, the nanobodies of the present invention can be made into a nasal spray formulation and easily pass through the nasal epithelial cells of inflammatory patients to efficiently reduce or eliminate the inflammatory response.

Having described specific embodiment of the present invention in detail above, it is to be understood that variants and modifications thereof falling within the spirit of the invention may become apparent to those skilled in this art, and the scope of this invention is to be determined by appended claims and their equivalents.

**References**

**EP 4 717 710 A1**

1. Zimmermann, I. et al. Synthetic single domain antibodies for the conformational trapping of membrane proteins. eLife 7, e34317 (2018).

2. Bhattacharjee, N. & Biswas, P. Position-specific propensies of amino acids in the b-strand. 10 (2010).

3. Zahnd, C., Amstutz, P. & Pluckthun, A. Ribosome display: selecting and evolving proteins in vitro that specifically bind to a target. Nat. Methods 4, 269-279 (2007).

4. Zimmermann, I. et al. Generation of synthetic nanobodies against delicate proteins. Nat. Protoc. 15, 1707-1741 (2020).

5. Geertsma, E. R. & Dutzler, R. A Versatile and Efficient High-Throughput Cloning Tool for Structural Biology. Biochemistry 50, 3272-3278 (2011).

6. Mikita, T., Campbell, D., Wu, P., Williamson, K. & Schindler, U. Requirements for interleukin-4-induced gene expression and functional characterization of Stat6. Mol. Cell. Biol. 16, 5811-5820 (1996).

7. Yu, X. et al. A robust reporter assay for the determination of the bioactivity of IL-4R-targeted therapeutic antibodies. J. Pharm. Biomed. Anal. 199, 114033 (2021).

## Claims

1. An anti-IL-4R antibody or antigen-binding fragment thereof comprising the amino acid sequence of SEQ ID NO: 1, wherein the anti-IL-4R antibody or antigen-binding fragment thereof comprises substitutions of one or more amino acid residues selected from the group consisting of the 5th residue and the 11th residue in the amino acid sequence of SEQ ID NO: 1.

2. The anti-IL-4R antibody or antigen-binding fragment thereof of claim 1, wherein the substitution of the 5th residue is a substitution to Arg.

3. The anti-IL-4R antibody or antigen-binding fragment thereof of claim 1, wherein the substitution of the 11th residue is a substitution to Tyr.

4. The anti-IL-4R antibody or antigen-binding fragment thereof of claim 1, wherein the antibody or antigen-binding fragment thereof comprises one or more amino acid sequences selected from the group consisting of SEQ ID NO:2, SEQ ID NO:3 and SEQ ID NO:4.

5. An anti-IL-4R antibody or antigen-binding fragment thereof comprising the amino acid sequence of SEQ ID NO: 5, wherein the anti-IL-4R antibody or antigen-binding fragment thereof comprises substitutions of one or more amino acid residues selected from the group consisting of the 104th residue and the 110th residue in the amino acid sequence of SEQ ID NO: 5.

6. The anti-IL-4R antibody or antigen-binding fragment thereof of claim 5, wherein the substitution of the 104th residue is a substitution to Arg.

7. The anti-IL-4R antibody or antigen-binding fragment thereof of claim 5, wherein the substitution of the 110th residue is a substitution to Tyr.

8. The anti-IL-4R antibody or antigen-binding fragment thereof of claim 5, wherein the anti-IL-4R antibody or antigen-binding fragment thereof comprises one or more amino acid sequences selected from the group consisting of SEQ ID NO: 6, SEQ ID NO: 7, and SEQ ID NO: 8.

9. The antibody or antigen-binding fragment thereof of any one of claims 1 to 8, wherein the antigen-binding fragment is a nanobody.

10. The anti-IL-4R antibody or antigen-binding fragment thereof of claim 9, wherein the nanobody comprises a convex scaffold structure.

11. A dimeric antibody or antigen-binding fragment thereof comprising: two antibodies or antigen-binding fragments thereof that are identical or different from each other, wherein each antibody or antigen-binding fragment thereof is selected from the group consisting of SEQ ID NOs: 1, 2, 3, 4, 5, 6, 7 and 8; and a linker.

12. The dimeric antibody or antigen-binding fragment thereof of claim 11, wherein the dimeric antibody or antigen-binding fragment thereof consists of two antibodies or antigen-binding fragments thereof having the amino acid sequence of SEQ ID NO: 5 and a linker connecting the two antibodies or antigen-binding fragments thereof.

13. The dimeric antibody or antigen-binding fragment thereof of claim 11, wherein the dimeric antibody or antigen-binding fragment thereof consists of two antibodies or antigen-binding fragments thereof having the amino acid sequence of SEQ ID NO: 6 and a linker connecting the two antibodies or antigen-binding fragments thereof.

14. The dimeric antibody or antigen-binding fragment thereof of claim 11, wherein the dimeric antibody or antigen-binding fragment thereof consists of two antibodies or antigen-binding fragments thereof having the amino acid sequence of SEQ ID NO: 7 and a linker connecting the two antibodies or antigen-binding fragments thereof.

15. The dimeric antibody or antigen-binding fragment thereof of claim 11, wherein the dimeric antibody or antigen-binding fragment thereof consists of two antibodies or antigen-binding fragments thereof having the amino acid sequence of SEQ ID NO: 8 and a linker connecting the two antibodies or antigen-binding fragments thereof.

16. The dimeric antibody or antigen-binding fragment thereof of any one of claims 11 to 15, wherein the linker consists of three amino acids.

17. The dimeric antibody or antigen-binding fragment thereof of claim 16, wherein the linker is a peptide having sequence of SEQ ID NO: 9.

18. A nucleic acid molecule encoding the antibody or antigen-binding fragment thereof of any one of claims 1, 5 or 11.

19. A pharmaceutical composition for preventing or treating an inflammatory disease or an autoimmune disease, comprising the antibody or antigen-binding fragment thereof of any one of claims 1, 5, or 11, or the nucleic acid molecule of claim 18, as an active ingredient.

20. The composition of claim 19, wherein the inflammatory disease or autoimmune disease is selected from group consisting of rhinitis, conjunctivitis, periodontitis, otitis media, sore throat, tonsillitis, pneumonia, gastric ulcer, gastritis, Crohn's disease, colitis, hemorrhoids, gout, ankylosing spondylitis, rheumatic fever, rheumatoid arthritis, rheumatoid polymyalgia, lupus, fibromyalgia, psoriatic arthritis, osteoarthritis, periarthritis of the shoulder, tendonitis, tenosynovitis, periarthritis, myositis, polymyositis, dermatomyositis, hepatitis, cystitis, nephritis, Sjogren's syndrome, multiple sclerosis, inflammatory bowel disease, asthma, type 1 diabetes, psoriasis, eczema, dermatosclerosis, vitiligo, peripheral neuritis, uveitis, autoimmune thrombocytopenia, autoimmune myocarditis, atopic dermatitis, primary liver cirrhosis, dry eye, Goodpasture's syndrome, autoimmune meningoencephalitis, Addison's disease, autoimmune parotitis, dystrophic bullous epidermolysis, epididymitis, glomerulonephritis, Graves' disease, celiac disease, Guillain-Barré syndrome, Hashimoto's disease, hemolytic anemia, myasthenia gravis, amyotrophic lateral sclerosis, pemphigus vulgaris, sarcoidosis, spondyloarthropathy, thyroiditis, vasculitis, myxedema, pernicious anemia, antiphospholipid syndrome, and graft-versus-host disease.

21. The composition of claim 19, wherein the rhinitis is nasal polyps or sinusitis.

22. The composition of claim 21, wherein the sinusitis is Chronic Sinusitis (CRS).

23. The composition of claim 22, wherein the chronic sinusitis is chronic sinusitis with nasal polyps (CRSwNP).

24. The composition of claim 19, wherein the antibody or antigen-binding fragment thereof, or the nucleic acid molecule is administered intranasally.

**FIG. 1**

**FIG. 2**

**FIG. 3**

FIG. 4

Reporter assay

FIG. 5

HEK293 Luciferase reporter cell
Binding assay

**FIG. 6a**

No treatment

IL4 only

DAPI
FOXJ1

IL4 + Dupilumab
25 ug/ml

IL4 + H5
5 ug/ml

IL4 + Dupilumab
50 ug/ml

IL4 + H5
10 ug/ml

**FIG. 6b**

DAPI
FOXJ1

No treatment

IL4 only

IL4 + Dupilumab
25 ug/ml

IL4 + H5
5 ug/ml

IL4 + Dupilumab
50 ug/ml

IL4 + H5
10 ug/ml

FIG. 6c

Dupilumab only

H5 only

DAPI
FOXJ1

IL4 + MBP Sb
5 ug/ml

IL4 + MBP Sb
10 ug/ml

**FIG. 6d**

Dupilumab only

H5 only

DAPI
FOXJ1

IL4 + MBP Sb
5 ug/ml

IL4 + MBP Sb
10 ug/ml

**FIG. 7**

**FIG. 8**

H5 E104R
KD: 1.06 uM

H5 A110Y
KD: 708 nM

H5
KD: 1.42 uM

15.625nM
31.25nM
62.5nM
125nM
250nM
500nM
1000nM
2000nM

Time(s)

RU

**FIG. 9a**

Cell binding

**FIG. 9b**

IL-4 reporter assay

**FIG. 9c**

**FIG. 10**

**FIG. 11**

**FIG. 12a**

**FIG. 12b**

IL-4 reporter assay

**FIG. 12c**

IL-13 reporter assay

**FIG. 13**

Apical side

Basal side

Human nasal epithelial cell

Micro-pore membrane

Culture medium

Goblet cell

Ciliated cell

Secretory cell

Basal cell

MUC5AC expression · CCL26 expression · FOXJ1 expression

FIG. 14a

EP 4 717 710 A1

**FIG. 14b**

FIG. 15

IL-4

z

z

x

y

No treatment

IL-4 only

Dupilumab 100 μg/ml

H5 10 μg/ml

I

z

z

x

y

No treatment

IL-13 only

IL-13

Dupilumab 1 μg/ml

Dupilumab 10 μg/ml

H5 0.1 μg/ml

H5 1 μg/ml

DAPI
MUC5AC

EP 4 717 710 A1

35

**FIG. 16**

Blot: anti-His, anti-human IgG

**FIG. 17**

Band intensity

FIG. 18

ZO-1 expression

Occludin expression

## INTERNATIONAL SEARCH REPORT

| International application No. |
| --- |
| **PCT/KR2024/006903** |

| A. | CLASSIFICATION OF SUBJECT MATTER |
| --- | --- |

**C07K 16/28**(2006.01)i; **A61P 29/00**(2006.01)i; **A61P 37/00**(2006.01)i; **A61P 11/02**(2006.01)i; **A61K 39/00**(2006.01)i

According to International Patent Classification (IPC) or to both national classification and IPC

| B. | FIELDS SEARCHED |
| --- | --- |

Minimum documentation searched (classification system followed by classification symbols)

C07K 16/28(2006.01); A61K 39/00(2006.01); A61K 39/395(2006.01); A61P 17/00(2006.01); A61P 29/00(2006.01); C12N 15/13(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: IL-4R, 항체(antibody), 나노바디(nanobody), 이량체(dimer), 염증성 질환 (inflammatory disease), 자가면역 질환(autoimmune disease)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | CN 111690066 A (NANJING RONGJIEKANG BIOTECHNOLOGY CO., LTD.) 22 September 2020 (2020-09-22)<br>See claims 1-14. | 1-18 |
| A | US 2022-0056143 A1 (INTERVET INC.) 24 February 2022 (2022-02-24)<br>See claims 25-38. | 1-18 |
| A | KR 10-2022-0091566 A (SHANGHAI NOVAMAB BIOPHARMACEUTICALS CO., LTD.) 30 June 2022 (2022-06-30)<br>See claims 1-10; and paragraph [0169]. | 1-18 |
| A | KR 10-2023-0004576 A (SHANGHAI MABGEEK BIOTECH. CO., LTD.) 06 January 2023 (2023-01-06)<br>See claims 1-15. | 1-18 |
| A | KR 10-2011-0074980 A (REGENERON PHARMACEUTICALS, INC.) 05 July 2011 (2011-07-05)<br>See claims 1-16; and paragraph [0018]. | 1-18 |

☐ Further documents are listed in the continuation of Box C.　　☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **04 September 2024** | **04 September 2024** |

| Name and mailing address of the ISA/KR | Authorized officer |
| --- | --- |
| **Korean Intellectual Property Office**<br>**Government Complex-Daejeon Building 4, 189 Cheongsa-**<br>**ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/006903**

| Box No. I | Nucleotide and/or amino acid sequence(s) (Continuation of item 1.c of the first sheet) |
| --- | --- |

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2. ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/006903** |

| **Box No. II**      **Observations where certain claims were found unsearchable (Continuation of item 2 of first sheet)** |
| --- |

This international search report has not been established in respect of certain claims under Article 17(2)(a) for the following reasons:

1. ☐    Claims Nos.:
   because they relate to subject matter not required to be searched by this Authority, namely:

2. ☑    Claims Nos.: **20-24**
   because they relate to parts of the international application that do not comply with the prescribed requirements to such an extent that no meaningful international search can be carried out, specifically:

   Claims 20-24 refer to claims violating the manner of referring to dependent claims (PCT Rule 6.4(a)), and thus are unclear.

3. ☑    Claims Nos.: **19**
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

| International application No. |
| --- |
| **PCT/KR2024/006903** |

| Patent document cited in search report | | | Publication date (day/month/year) | Patent family member(s) | | | Publication date (day/month/year) |
| --- | --- | --- | --- | --- | --- | --- | --- |
| CN | 111690066 | A | 22 September 2020 | CN | 111690066 | B | 22 July 2022 |
| | | | | CN | 114957472 | A | 30 August 2022 |
| | | | | CN | 114957472 | B | 31 October 2023 |
| | | | | CN | 117327181 | A | 02 January 2024 |
| US | 2022-0056143 | A1 | 24 February 2022 | AU | 2016-239858 | A1 | 12 October 2017 |
| | | | | AU | 2016-239858 | B2 | 01 July 2021 |
| | | | | CA | 2980087 | A1 | 06 October 2016 |
| | | | | CA | 3005696 | A1 | 22 June 2017 |
| | | | | CN | 107683291 | A | 09 February 2018 |
| | | | | CN | 107683291 | B | 19 November 2021 |
| | | | | CN | 113861294 | A | 31 December 2021 |
| | | | | CN | 113861294 | B | 08 March 2024 |
| | | | | EP | 3277722 | A1 | 07 February 2018 |
| | | | | EP | 3277722 | B1 | 18 August 2021 |
| | | | | EP | 3390451 | A1 | 24 October 2018 |
| | | | | EP | 3954710 | A2 | 16 February 2022 |
| | | | | EP | 3954710 | A3 | 20 July 2022 |
| | | | | JP | 2018-511322 | A | 26 April 2018 |
| | | | | JP | 2019-506146 | A | 07 March 2019 |
| | | | | JP | 2021-191779 | A | 16 December 2021 |
| | | | | JP | 2024-010002 | A | 23 January 2024 |
| | | | | JP | 6938383 | B2 | 22 September 2021 |
| | | | | JP | 7008020 | B2 | 25 January 2022 |
| | | | | JP | 7371070 | B2 | 30 October 2023 |
| | | | | US | 10858437 | B2 | 08 December 2020 |
| | | | | US | 11091556 | B2 | 17 August 2021 |
| | | | | US | 2018-0346580 | A1 | 06 December 2018 |
| | | | | US | 2018-0371097 | A1 | 27 December 2018 |
| | | | | US | 2021-0040223 | A1 | 11 February 2021 |
| | | | | WO | 2016-156588 | A1 | 06 October 2016 |
| | | | | WO | 2017-102920 | A1 | 22 June 2017 |
| KR | 10-2022-0091566 | A | 30 June 2022 | AU | 2020-377355 | A1 | 06 May 2021 |
| | | | | CA | 3156084 | A1 | 06 May 2021 |
| | | | | CN | 111825766 | A | 27 October 2020 |
| | | | | CN | 111825766 | B | 11 May 2021 |
| | | | | EP | 4053161 | A1 | 07 September 2022 |
| | | | | JP | 2023-501182 | A | 18 January 2023 |
| | | | | JP | 7387206 | B2 | 28 November 2023 |
| | | | | US | 12012456 | B2 | 18 June 2024 |
| | | | | US | 2022-0411519 | A1 | 29 December 2022 |
| | | | | WO | 2021-082573 | A1 | 06 May 2021 |
| KR | 10-2023-0004576 | A | 06 January 2023 | AU | 2021-255445 | A1 | 21 October 2021 |
| | | | | CA | 3175786 | A1 | 21 October 2021 |
| | | | | CN | 111598131 | A | 28 August 2020 |
| | | | | CN | 111598131 | B | 25 August 2023 |
| | | | | CN | 112092972 | A | 18 December 2020 |
| | | | | CN | 112092972 | B | 04 June 2024 |
| | | | | CN | 113527485 | A | 22 October 2021 |
| | | | | CN | 115461368 | A | 09 December 2022 |
| | | | | EP | 3819824 | A2 | 12 May 2021 |

Form PCT/ISA/210 (patent family annex) (July 2022)

## INTERNATIONAL SEARCH REPORT
### Information on patent family members

International application No.

**PCT/KR2024/006903**

| Patent document cited in search report | | | Publication date (day/month/year) | Patent family member(s) | | | Publication date (day/month/year) |
|---|---|---|---|---|---|---|---|
| | | | | EP | 3819824 | A3 | 29 September 2021 |
| | | | | EP | 4137517 | A1 | 22 February 2023 |
| | | | | JP | 2021-103558 | A | 15 July 2021 |
| | | | | JP | 2023-515260 | A | 12 April 2023 |
| | | | | JP | 7177878 | B2 | 24 November 2022 |
| | | | | JP | 7454882 | B2 | 25 March 2024 |
| | | | | KR | 10-2487260 | B1 | 10 January 2023 |
| | | | | US | 11727676 | B2 | 15 August 2023 |
| | | | | US | 11939387 | B2 | 26 March 2024 |
| | | | | US | 2021-0232856 | A1 | 29 July 2021 |
| | | | | US | 2023-0203172 | A1 | 29 June 2023 |
| | | | | US | 2024-0190979 | A1 | 13 June 2024 |
| | | | | WO | 2021-208327 | A1 | 21 October 2021 |
| | | | | WO | 2021-208881 | A1 | 21 October 2021 |
| KR | 10-2011-0074980 | A | 05 July 2011 | AU | 2007-314522 | A1 | 08 May 2008 |
| | | | | AU | 2009-311496 | A1 | 14 May 2010 |
| | | | | CA | 2664343 | A1 | 08 May 2008 |
| | | | | CA | 2737044 | A1 | 14 May 2010 |
| | | | | CN | 101522716 | A | 02 September 2009 |
| | | | | CN | 102197052 | A | 21 September 2011 |
| | | | | CN | 103739711 | A | 23 April 2014 |
| | | | | CN | 106267190 | A | 04 January 2017 |
| | | | | EP | 2069403 | B1 | 07 May 2014 |
| | | | | EP | 2636685 | A1 | 11 September 2013 |
| | | | | EP | 2769992 | B1 | 30 December 2020 |
| | | | | EP | 3064511 | A1 | 07 September 2016 |
| | | | | EP | 3351560 | A1 | 25 July 2018 |
| | | | | JP | 2012-507294 | A | 29 March 2012 |
| | | | | JP | 2020-007378 | A | 16 January 2020 |
| | | | | JP | 2024-040526 | A | 25 March 2024 |
| | | | | JP | 5291802 | B2 | 18 September 2013 |
| | | | | JP | 7100731 | B2 | 13 July 2022 |
| | | | | JP | 7442581 | B2 | 04 March 2024 |
| | | | | KR | 10-1474227 | B1 | 18 December 2014 |
| | | | | KR | 10-1599706 | B1 | 04 March 2016 |
| | | | | KR | 10-2009-0088865 | A | 20 August 2009 |
| | | | | US | 2010-0021476 | A1 | 28 January 2010 |
| | | | | US | 2014-0271681 | A1 | 18 September 2014 |
| | | | | US | 2018-0179288 | A1 | 28 June 2018 |
| | | | | US | 2021-0163611 | A1 | 03 June 2021 |
| | | | | US | 7605237 | B2 | 20 October 2009 |
| | | | | US | 8735095 | B2 | 27 May 2014 |
| | | | | WO | 2008-054606 | A2 | 08 May 2008 |
| | | | | WO | 2008-054606 | A3 | 05 February 2009 |
| | | | | WO | 2010-053751 | A1 | 14 May 2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8810649 A
- WO 88106630 A
- WO 8807085 A
- WO 8807086 A
- WO 8809344 A

### Non-patent literature cited in the description

- **H. NEURATH** ; **R.L. HILL**. The Proteins. Academic Press, 1979
- **HUANG et al.** *Comp. Appl. BioSci.*, 1992, vol. 8, 155-65
- **PEARSON et al.** *Meth. Mol. Biol.*, 1994, vol. 24, 307-31
- **UHLMAN** ; **PEYMAN**. *Chemical Reviews*, 1990, vol. 90, 543-584
- **ZIMMERMANN, I. et al.** Synthetic single domain antibodies for the conformational trapping of membrane proteins. *eLife*, 2018, vol. 7, e34317
- **BHATTACHARJEE, N.** ; **BISWAS, P.** *Position-specific propensities of amino acids in the b-strand*, 2010, vol. 10
- **ZAHND, C** ; **AMSTUTZ, P.** ; **PLUCKTHUN, A.** Ribosome display: selecting and evolving proteins in vitro that specifically bind to a target. *Nat. Methods*, 2007, vol. 4, 269-279
- **ZIMMERMANN, I. et al.** Generation of synthetic nanobodies against delicate proteins. *Nat. Protoc.*, 2020, vol. 15, 1707-1741
- **GEERTSMA, E. R.** ; **DUTZLER, R.** A Versatile and Efficient High-Throughput Cloning Tool for Structural Biology. *Biochemistry*, 2011, vol. 50, 3272-3278
- **MIKITA, T** ; **CAMPBELL, D.** ; **WU, P.** ; **WILLIAMSON, K.** ; **SCHINDLER, U.** Requirements for interleukin-4-induced gene expression and functional characterization of Stat6.. *Mol. Cell. Biol.*, 1996, vol. 16, 5811-5820
- **YU, X. et al.** A robust reporter assay for the determination of the bioactivity of IL-4R-targeted therapeutic antibodies. *J. Pharm. Biomed. Anal.*, 2021, vol. 199, 114033